# EUROPEAN PATENT SPECIFICATION

(11) **EP 1 713 511 B1**
(45) Date of publication and mention of the grant of the patent: **09.11.2011**
(21) Application number: 05721759.8
(22) Date of filing: 10.01.2005
(51) Int. Cl.: A61K 48/00, A61P 35/00

(54) **THERAPEUTIC AGENT FOR TREATMENT OF CANCER COMPRISING HUMAN APOLIPOPROTEIN (A) KRINGLES LK68 OR LK8 GENES AS EFFECTIVE INGREDIENT, AND METHOD FOR TREATING CANCER USING THE SAME**
THERAPEUTISCHES MITTEL ZUR BEHANDLUNG VON KREBS MIT HUMANEN APOLIPOPROTEIN (A) KRINGLES LK68 ODER LK8 GENEN ALS WIRKSAME INHALTSSTOFFE, UND VERFAHREN ZUR BEHANDLUNG VON KREBS DAMIT
AGENT THERAPEUTIQUE POUR LE TRAITEMENT DU CANCER COMPORTANT UN GENE LK68 OU LK8 DES KRINGLES DE L'APOLIPOPROTEINE (A) EN TANT QUE PRINCIPE ACTIF, ET PROCEDE DE TRAITEMENT DU CANCER UTILISANT CET AGENT THERAPEUTIQUE

(30) Priority: 09.01.2004 KR 2004001695
(43) Date of publication of application: 25.10.2006
(73) Proprietor: Mogam Biotechnology Research Institute, Yongin-si, 449-910 Gyeonggi-do (KR)
(72) Inventor: LEE, Kyuhyun, Seoul 139-220 (KR); YU, Hyunkyung, Gyunggi-Do 441-480 (KR); LEE, Ho-Jeong, Suji-eup Yongin, Gyunggi-Do 449840 (KR); AHN, Jin-Hyung, Gyunggi-Do 449-846 (KR); KIM, Jang-Seong, Paldal-ku Suwon Gyunggi-Do 442-400 (KR); JO, Eui-Cheol, Suwon City Gyunggi-Do 443-470 (KR); YOON, Yeup, Gyunggi-Do 427-010 (KR); PARK, Doo-Hong, Seoul 137-062 (KR); YUN, Seong-Tae, Gyunggi-Do 449-160 (KR)
(74) Representative: Grünecker, Kinkeldey, Stockmair & Schwanhäusser
(86) International application number: PCT/KR2005/000075
(87) International publication number: WO 2005/065720

(56) References cited:
- WO-A1-01/19868
- WO-A1-2004/073730
- US-A1- 2002 164 717
- US-A1- 2004 259 202
- SAIMURA MICHIYO ET AL: "Intraperitoneal injection of adenovirus-mediated NK4 gene suppresses peritoneal dissemination of pancreatic cancer cell line AsPC-1 in nude mice" CANCER GENE THERAPY, NORWALK, CT, US, vol. 9, no. 10, October 2002 (2002-10), pages 799-806, XP002246640 ISSN: 0929-1903
- YU ET AL: 'Suppression of colorectal cancer liver metastasis and extension of survival by expression of apolopoprotein(a) kringles' CANCER RESEARCH vol. 64, October 2004, pages 7092 - 7098, XP002450346
- LOGRASSO ET AL: 'Cloning, expression, and characterization of human apolipoprotein (a) kringle IV37' THE JOURNAL OF BIOLOGICAL CHEMISTRY vol. 269, no. 34, August 1994, pages 21820 - 21827, XP002909804

### sequence Listing

<110> MOGAM BIOTECHNOLOGY RESEARCH INSTITUTE
<120> THERAPEUTIC AGENT FOR TREATMENT OF CANCER COMPRISING HUMAN APOLIPOPROTEIN (A) KRINGLES LK68 OR LK8 GENES AS EFFECTIVE INGREDIENT, AND METHOD FOR TREATING CANCER USING THE SAME
<130> EP43275HV091pau
<140> EP 05 721 759.8
   <141> 2006-07-18
<150> PCT/KR2005/00075
   <151> 2005-01-10
<150> KR1020040001695
   <151> 2004-01-09
<160> 11
<170> KopatentIn 1.71
<210> 1
   <211> 924
   <212> DNA
   <213> Homo sapiens
<400> 1
   aaaagccctg tggtccagga ttgctaccat ggtgatggac ggagttatcg aggcatatcc 60
   tccaccactg tcacaggaag gacctgtcaa tcttggtcat ctatgatacc acactggcat 120
   cagaggaccc cagaaaacta cccaaatgct ggcctgaccg agaactactg caggaatcca 180
   gattctggga aacaaccctg gtgttacaca accgatccgt gtgtgaggtg ggagtactgc 240
   aatctgacac aatgctcaga aacagaatca ggtgtcctag agactcccac tgttgttcca 300
   gttccaagca tggaggctca ttctgaagca gcaccaactg agcaaacccc tgtggtccgc 360
   cagtgctacc atggcaatgg ccagagttat cgaggcacat tctccaccac tgtcacagga 420
   aggacatgtc aatcttggtc atccatgaca ccacaccggc atcagaggac cccagaaaac 480
   tacccaaatg atggcctgac aatgaactac tgcaggaatc cagatgccga tacaggccct 540
   tggtgtttta ccacggaccc cagcatcagg tgggagtact gcaacctgac gcgatgctca 600
   gacacagaag ggactgtggt cgctcctccg actgtcatcc aggttccaag cctagggcct 660
   ccttctgaac aagactgtat gtttgggaat gggaaaggat accggggcaa gaaggcaacc 720
   actgttactg ggacgccatg ccaggaatgg gctgcccagg agccccatag acacagcacg 780
   ttcattccag ggacaaataa atgggcaggt ctggaaaaaa attactgccg taaccctgat 840
   ggtgacatca atggtccctg gtgctacaca atgaatccaa gaaaactttt tgactactgt 900
   gatatccctc tctgtgcatc ctct 924
<210> 2
   <211> 258
   <212> DNA
   <213> Homo sapiens
<400> 2
   gaacaggact gcatgtttgg gaatgggaaa ggataccggg gcaagaaggc aaccactgtt 60
   actgggacgc catgccagga atgggctgcc caggagcccc atagacacag cacgttcatt 120
   ccagggacaa ataaatgggc aggtctggaa aaaaattact gccgtaaccc tgatggtgac 180
   atcaatggtc cctggtgcta cacaatgaat ccaagaaaac tttttgacta ctgtgatatc 240
   cctctctgtg catcctct 258
<210> 3
   <211> 39
   <212> DNA
   <213> Artificial sequence
<220>
   <223> sense primer of LK68
<400> 3
   gcggcccagc cggccaaaag ccctgtggtc caggattgc 39
<210> 4
   <211> 33
   <212> DNA
   <213> Artificial Sequence
<220>
   <223> sense primer of LK8
<400> 4
   gcggcccagc cggcccctcc ttctgaacaa gac 33
<210> 5
   <211> 36
   <212> DNA
   <213> Artificial sequence
<220>
   <223> antisense primer of LK68 or LK8
<400> 5
   ccgctcgagt taagaggatg cacagagagg gatatc 36
<210> 6
   <211> 39
   <212> DNA
   <213> Artificial sequence
<220>
   <223> sense primer for virus vector
<400> 6
   ccggaattcc tagccaccat ggagacagac acactcctg 39
<210> 7
   <211> 29
   <212> DNA
   <213> Artificial sequence
<220>
   <223> LK68 NdeI sense primer
<400> 7
   tccatatgaa aagccctgtg gtccaggat 29
<210> 8
   <211> 23
   <212> DNA
   <213> Artificial sequence
<220>
   <223> LK68 BamHI antisense primer
<400> 8
   cgggatcctt aagaggatgc aca 23
<210> 9
   <211> 24
   <212> DNA
   <213> Artificial sequence
<220>
   <223> LK8 Ndel sense primer
<400> 9
   catatgtctg aacaagactg tatg 24
<210> 10
   <211> 21
   <212> DNA
   <213> Artificial sequence
<220>
   <223> IgK EcoRI forward primer
<400> 10
   ggaattcaag ctggctagcc a 21
<210> 11
   <211> 50
   <212> DNA
   <213> Artificial Sequence
<220>
   <223> LK68 Flag epitope, XhoI antisense primer
<400> 11
   atctcgagtt acttgtcatc gtcgtccttg tagtcagagg atgcacagag 50

## Description

### Technical Field

The present invention relates to an anticancer agent for gene therapy, more precisely, an anticancer or an anti-metastatic agent for gene therapy comprising a gene carrier or cells containing human apolipoprotein(a) kringle gene, and a method for the treatment of cancer by using the same.

### Background Art

A tumor is developed by uncontrollable disordered abnormal cell proliferation. If this tumor shows a destructive growth, invasiveness and metastasis, it is regarded as a malignant cancer. Invasiveness is a character to infiltrate or destroy surrounding tissues, and in particular, a basal layer forming a boundary of tissues is destroyed by the character, resulting in the local spread and sometimes inflow of a tumor through circulatory system. Metastasis means the spread of tumor cells from the original birthplace to other areas through lymphatic or blood vessels. In a broad sense, metastasis also means the direct extension of tumor cells through serous body cavity or other space.

Surgical operation, radiotherapy and chemotherapy is widely used for the treatment of cancer singly or jointly. The surgical operation is a way to remove diseased tissues. Thus, tumors in specific regions such as breast, colon and skin can be effectively removed by the surgical operation. However, a tumor in vertebra or dispersive tumor like leukemia cannot be properly treated by the surgical operation.

Chemotherapy blocks cell replication or metabolism, and has been used for the treatment of breast cancer, lung cancer and testicular cancer. Though, patients with cancers who have been treated by chemotherapy have seriously suffered from the side effects of systemic chemotherapy. Motion sickness and vomiting are common but serious examples of all. The side effects of chemotherapy can even affect the life of a patient since they might drop the adaptability of a patient rapidly. Besides, DLT (Dose Limiting Toxicity) is also one of major side effects of chemotherapy, which draws a careful attention in the administration of a medicine. Mucositis is an example of DLT against anticancer agents such as 5-fluorouracil which is an antimetabolic cytotoxic agent, and methotrexate, and anticancer antibiotics like doxorubicin. If a patient suffers seriously from such side effects of chemotherapy, he or she should be hospitalized and given an anodyne for reducing pain. So, side effects of chemotherapy and radiotherapy are the biggest problem for the treatment of cancer patients.

Gene therapy is a method to treat or prevent diseases caused by the genetic variation in human cells, for example various genetic disorders, cancers, cardiovascular diseases, infective diseases, and autoimmune diseases, by taking advantage of DNA recombination technique, that is, a therapeutic gene is inserted into a patient to correct genetic defect or to promote or add functions of cells. More precisely, gene therapy is to treat a disease by sending a therapeutic gene to a target organ in order to induce the expression of therapeutic or normal protein in damaged cells. Gene therapy has advantages such as excellent specificity and improvement of recovery rate and speed, which are difficult to be regulated by other medicine, which enables long-term administration. Gene therapy is not for treating symptoms of a disease but for curing or eliminating the cause of the disease. For the success of the therapy, it is important to deliver a therapeutic gene to a target cell to improve its expression rate, which is essential technique in gene therapy.

A gene carrier is a necessary mediator for the insertion of a therapeutic gene to a target cell. An ideal gene carrier has to be no harmful for human, mass-produced, has to carry a gene to a target cell efficiently and has to express the gene continuously. Preparation of a gene carrier is a core technique in gene therapy. Most representative gene carriers widely used for gene therapy today are viral carriers such as adenovirus, adeno-associated virus, retrovirus and non-viral carriers such as liposome, polyethyleneamine, etc.

There are many ways to deliver a gene, for example, conjugation using liposome, methods using a receptor or virus as well as chemical or physical methods. Each method has merits and demerits. The present inventors selected a method using a viral carrier in consideration of delivery efficacy and expression efficiency. Among many viral carriers, adenovirus, adeno-associated virus and retrovirus have been most widely used.

Adenovirus is utilized for recombinant virus system in which E1 gene is defected and a therapeutic gene is inserted into the place. About 20% of vectors for gene therapy have been tried by the system (Journal of Gene Medicine Website, www.wiley.co.uk/genemd/clinical/). Adenovirus gene carrier infects various cells with ease but does not invade into chromosome of a host cell, so continuous expression cannot be expected but high expression within a short term is possible.

Adeno-associated virus contains a genome composed of ITR, *rep, cap* and ITR in that order. In order for adeno-associated virus to be growing, the aid of helper virus such as adenovirus and herpes virus is required. When adenovirus is used as a helper virus, E1, E2a, E4 and VA genes of adenovirus are necessary, so as to prepare a recombinant adeno-associated virus in which a therapeutic gene is inserted where *rep* and *cap* genes are defected (Samulski, R. J. et al., J. Virol., 63: 3822-3828, 1989). Adeno-associated virus shows continuous high expression because it enables insertion of a target gene into a specific region of adeno S1 of chromosome 19 of a host cell. Moreover, the virus cannot be growing by itself, so there has been no report on side effects accompanied with the use of the virus and no doubt on stability affected by the contamination with wild type virus, meaning that it is much safer than other systems.

As the most common vector system, retrovirus takes about 40% of total vectors used in clinical test for gene therapy (Journal of Gene Medicine Website, www.wiley.co.uk/genmed/clinical). A recombinant retrovirus is prepared by inserting a therapeutic gene and an antibiotics resistant gene like neomycin, hygromycin, or furomycin, in between LTR (long terminal repeat) of both ends, resembled to ITR (inverted terminal repeat) of adeno-associated virus (Miller, A.D. et al., Biotechniques, 7: 980-990, 1989). By the retrovirus system, a target gene can be inserted into chromosome of a host cell unspecifically and expressed continuously with high level. However, a mass-production of the virus is not expected and its infection rate toward non-proliferateve cells is too low. An indirect gene delivery is more preferred in the system than a direct gene insertion into a living body. That is, a recombinant virus is not directly injected into a living body, instead, cells cultured *in vitro* are infected with the recombinant virus containing a target gene first and then the cells transduced with the target gene are selected to be administrated into a living body. However, the forementioned system still has problems such as side effects by RCR (replication competent retrovirus) developed by the homologous recombination by a similar base sequence in host cells, insufficient yield of virus production or unsatisfactory infection efficiency.

As gene therapy strategies for controlling tumor cells, methods of using a tumor suppressor gene, using a replication-competent oncolytic virus, using a suicide gene and using an immunoregulatory gene, etc, have been used. The method of using a tumor suppressor gene is to treat cancer by delivering a tumor suppressor gene such as p53 into a patient specifically, where the gene is defected or deformed. In addition, the method of using a replication-competent oncolytic virus is to treat cancer by exploiting the damaged activity of tumor suppressor gene in tumor tissues by transferring a viral gene carrier that is able to be growing specifically in tumor cells to a human body. These two methods are the strategies to kill tumor cells directly. Alternately, the method of using a suicide gene is included in this category. A representative example of a suicide gene therapy is to treat disease by delivering a HSV-tk gene and chemical anticancer agents such as ganciclovir, which can induce death of tumor cells. On the contrary, the method to introduce an immunoregulatory gene is a kind of indirect treatment strategies, which carries one or more of the genes such as interleukin 12, interleukin 4, interleukin 7, gamma-interferon and tumor necrosis factor, etc, into a living body in order to provoke T cells to recognize tumor cells or induce apoptosis by blocking a tumor developing protein. On the other hand, the method to kill tumor cells by blocking nutrient supply by expressing angiogenesis inhibiting factors such as angiostatin or endostatin, etc, is also included in the category of indirect treatment strategies.

A kringle is a kind of a domain of a protein, which is composed of 80 amino acids and three intramolecular disulfide bonds. Kringle structure is found in many proteins such as angiostatin (O'Reilly, M.S. et al., Cell, 79:315-328, 1994), prothrombin (Walz, D.A. et al., Proc. Natl. Acad. Sci., 74:1069-1073, 1977), urokinase (Pennica, D. et al., Nature, 301:579-582, 1983), interstitial cell growth factor (Lukker, N.A. et al., Protein Eng., 7:895-903, 1994) and apolipoprotein(a) (McLean, J.W. et al., Nature, 330:132-137, 1987). A kringle is composed of an individual folding unit. The functions of the kringle have not been fully delineated yet, but considering the various functions of proteins having kringle structures, it is believed that the kringles do not have same functions.

Apolipoprotein(a), a kind of glycoprotein produced only in the liver, has kringle structures. Apolipoprotein(a) forms lipoprotein(a) by being covalently bound to apo B-100, a major protein component of low density lipoprotein (LDL) (Fless, G. M., J. Biol. Chem., 261: 8712-8717, 1986). Apolipoprotein(a) is responsible for the delivery of cholesterol *in vivo*. The increase of lipoprotein(a) level in blood serum itself is a major cause of artherosclerosis and heart disease (Armstrong, V.W. et al., Artherosclerosis, 62: 249-257, 1986; Assmann, G., Am. J. Cardiol., 77: 1179-1184, 1996). Apolipoprotein(a) is a mediator for the actions of lipoprotein(a). Apolipoprotein(a) includes two types of kringle regions which are very similar to kringle IV and V of plasminogen, and an inactive protease-like region. Apolipoprotein(a) kringle IV-like region is divided into 10 subtypes (subtype; IV1 ∼ IV10) according to homology of amino acid sequence, and each of them has only one copy except IV2 kringle which has 3 ∼ 42 copy numbers in various human alleles of apolipoprotein(a) gene. The last kringle V has 83.5% amino acid sequence homology with plasminogen kringle-5.

The present inventors have applied for a patent with an anticancer agent containing LK8 protein as an effective ingredient in 2003 (Korean Patent Application No.: 2003-10797). Solid tumor or metastatic tumor has long-term dormant stage, meaning it can be developed again after a long while from the treatment. However, an anticancer agent containing a protein as an effective ingredient has comparatively short half-life *in vivo.* Thus, in order to keep the anticancer effect, the required amount of protein has to be administered constantly to keep the level of anticancer protein in tumor cells.

The above method has many problems. First, it requires a huge amount of protein, for which facilities and techniques have to be equipped. Second, although the mass-production is possible, it costs a lot to both patients and producers. Third, long-term administration of a medicine is not easy at all for a patient. So, these problems have to be overcome prior to the development of an anticancer agent containing an anticancer protein as an effective ingredient.

Thus, the present inventors judged that gene therapy using a gene coding a protein is an effective alternative to overcome the problems of using a protein as an anticancer agent because it is able to keep the content of effective anticancer protein in systemic or local tumor. And the present inventors disclosed through animal tests using human apolipoprotein(a) kringle KIV9-KIV10-KV(LK68) and KV (LK8) genes could inhibit the growth of cancer and metastasis effectively. Finally, the present inventors completed this invention by confirming that LK68 and LK8 genes could be used as an anticancer agent.

### Disclosure

### Technical Problem

It is an object of the present invention to provide an anticancer or an anti-metastatic agent for gene therapy containing a gene carrier or cells containing human apolipoprotein(a) kringle KIV9-KIV10-KV(LK68) or KV (LK8) gene as an effective ingredient.

It is another object of the present invention to provide a method for the prevention or the treatment of a solid tumor, which includes a step of parenteral administration of a gene carrier or cells containing the LK68 or LK8 gene to an individual.

### Technical Solution

In order to achieve the above objects, the present invention provides an anticancer or an anti-metastatic agent for gene therapy containing a gene carrier or cells harboring human apolipoprotein(a) kringle KIV9-KIV10-KV(LK68) or KV(LK8) gene as an effective ingredient.

The present invention also provides a method for the prevention or the treatment of a solid tumor, which includes a step of parenteral administration of a gene carrier or cells containing the LK68 or LK8 gene to an individual.

### Description of Drawings

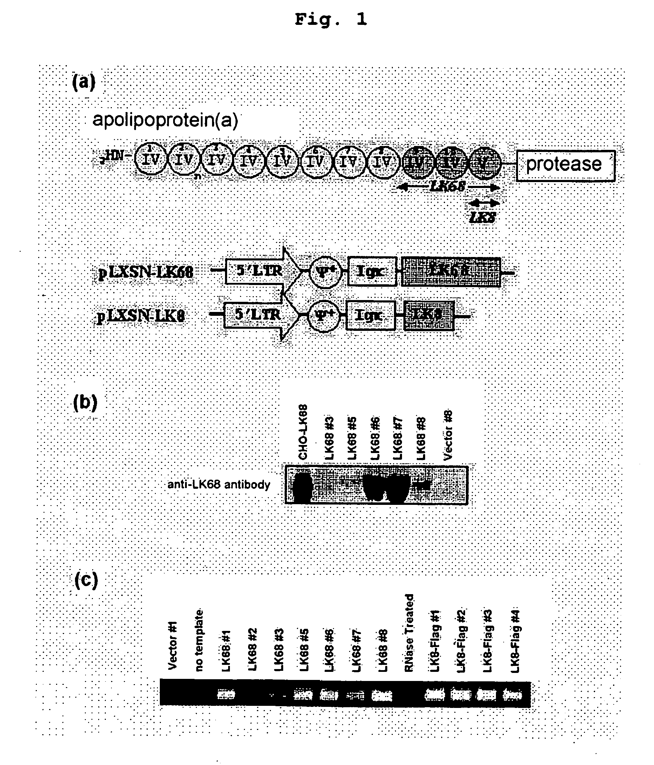

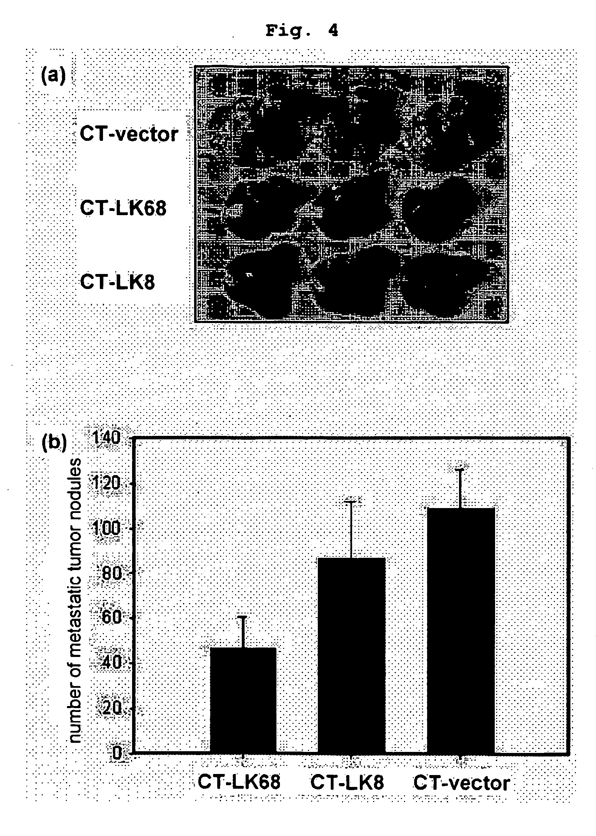

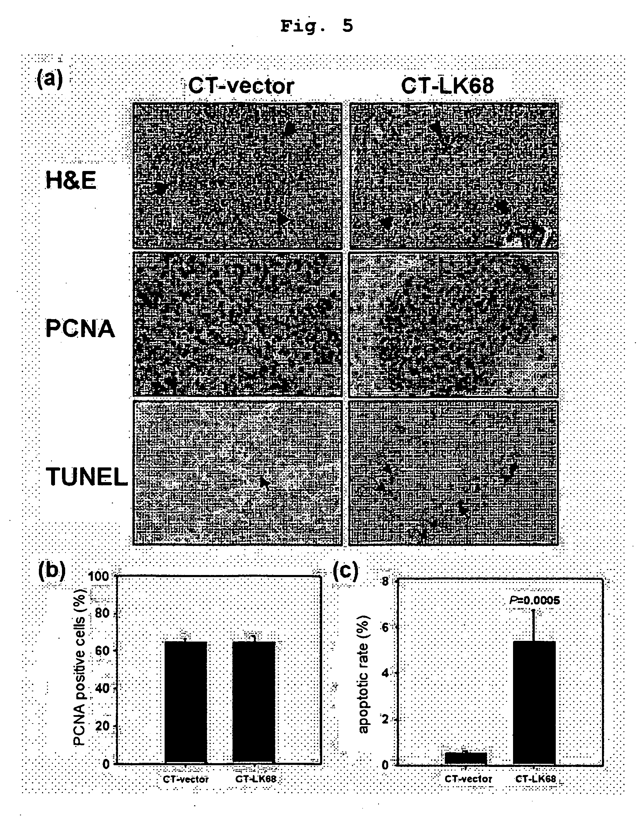

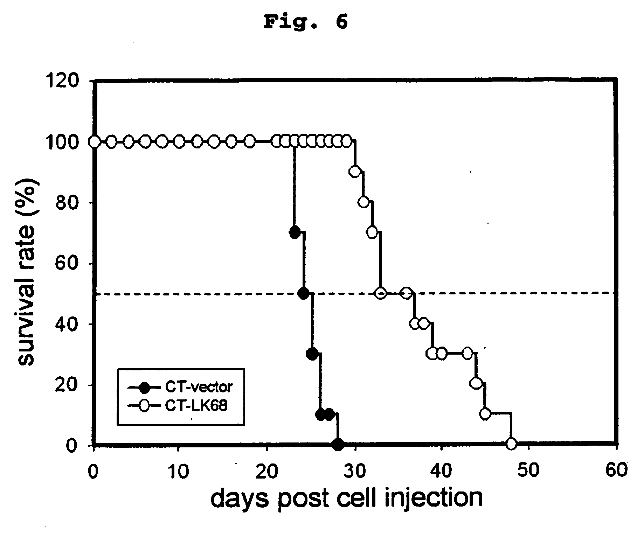

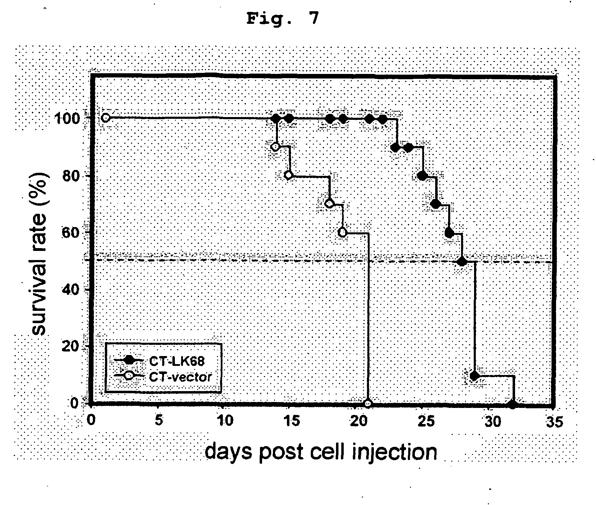

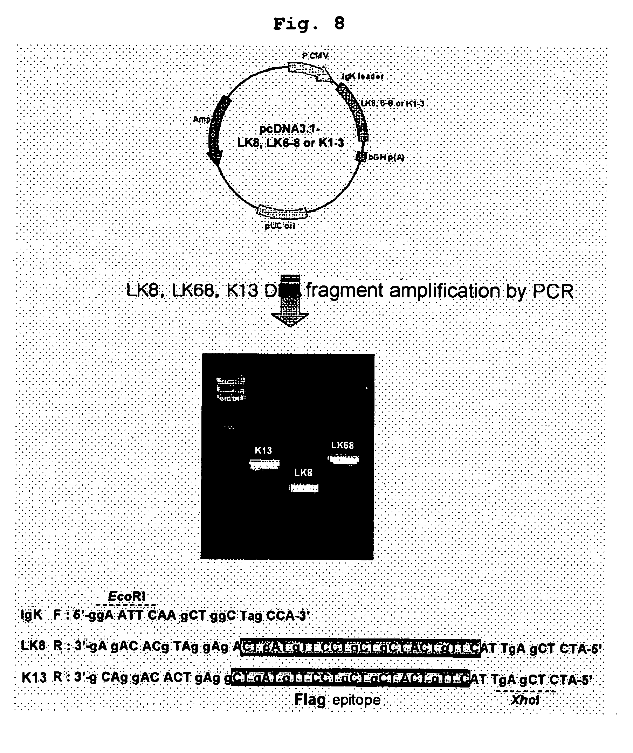

Fig. 1 is a schematic diagram and a set of photographs showing the preparation processes of colon carcinoma cell line where LK68 or LK8 gene has been introduced by using a recombinant virus. (a) a schematic diagram of vectors 'pLXSN-LK68' and 'pLXSN-LK8' for cloning of genes coding LK68 and LK8 proteins, (b) a photograph of Western blot analysis showing the expressions of LK68 and LK8 proteins in colon carcinoma cell line transfected with a recombinant virus, (c) a photograph of RT-PCR showing the expressions of LK68 and LK8 proteins in colon carcinoma cell line transfected with a recombinant virus.

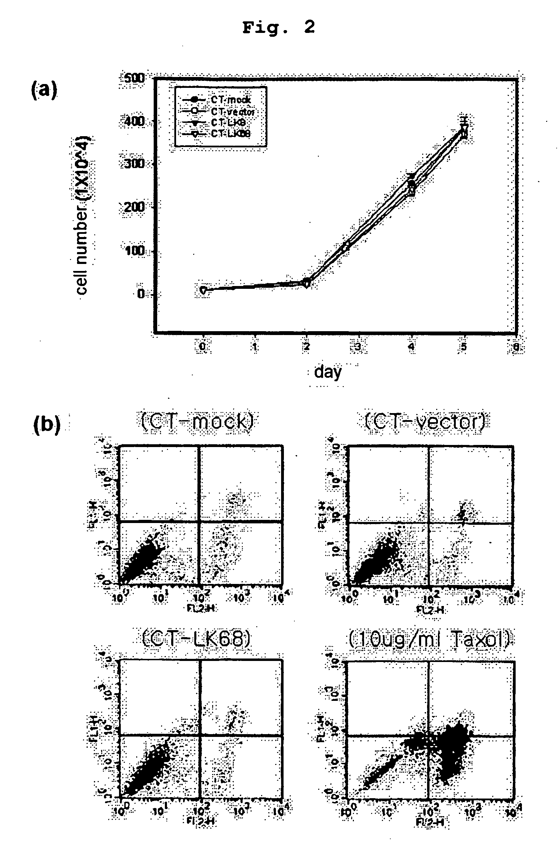

Fig. 2 is a set of graphs showing the growth of recombinant colon carcinoma cell line *in vitro,* which was measured by trypan blue staining (a) (in this graph, Y-axis presents cell number, and X-axis presents observation time), and the apoptosis of recombinant colon carcinoma cell line, which was observed by FACS (fluorescence-activated cell sorter; Becton Dickinson, San Jose, CA, USA) using annexin-V and PI (propidium iodide, Clontech) staining (b) (10 µg/ml of taxol was treated for positive control). The measurement and the observation were performed to investigate the effect of the introduction of LK68 or LK8 gene on the apoptosis and the growth of a target cell.
   CT-mock: Colon carcinoma cell line not infected with virus,
   CT-vector: Colon carcinoma cell line infected with plasmid without LK68 gene,
   CT-LK68: Colon carcinoma cell line infected with plasmid containing LK68 gene,
   10 µg/ml Taxol: CT-mock treated with taxol (Positive Control)

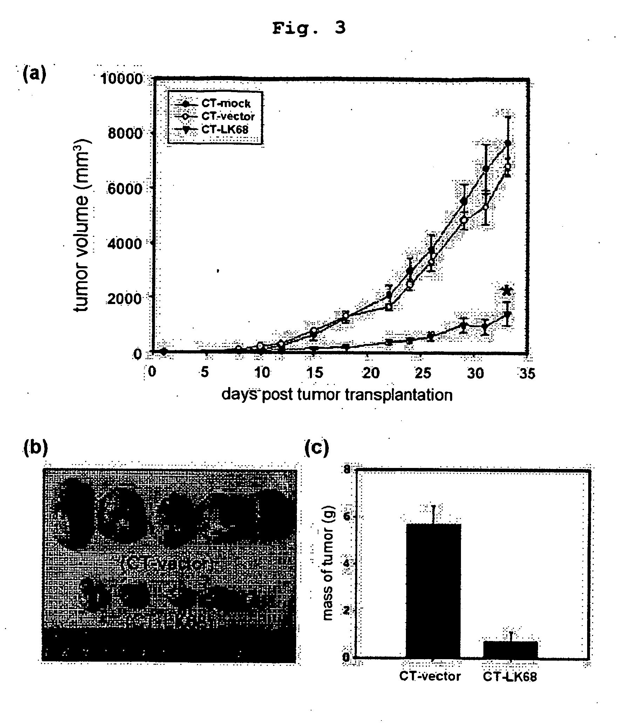

Fig. 3 is a set of photographs and graphs showing the growth of a solid tumor, which was measured after that each colon carcinoma cell line was injected into Balb/c nude mice (Charles River, Wilmington, MA, USA) hypodermically (a) (the statistic significance was examined by student's t-test (*: p < 0.0005), Y-axis presents the volume of a tumor calculated by the formula [length × (width)² × 0.52], X-axis presents days after tumor transplantation), the solid tumor separated from the mice on the final day of colon carcinoma cell line culture (b), and the weight of the solid tumor (c).
   CT-mock: Colon carcinoma cell line not infected with virus,
   CT-vector: Colon carcinoma cell line infected with plasmid without LK68 gene,
   CT-LK68: Colon carcinoma cell line infected with plasmid containing LK68 gene
Fig. 4 is a set of photographs and graphs showing the comparison of metastasis of colon carcinoma cells to liver (a) and the number of metastatic tumor nodules formed on the surface of liver (b). Colon carcinoma cells were transplanted in the spleen and metastasis to the liver was induced. Metastasis to the liver from the spleen was compared between experimental and control groups, in order to investigate whether or not LK68 or LK8 gene could inhibit metastasis after being introduced in colon carcinoma cells. And the inhibition of metastasis to the liver was investigated by counting the number of cancer cells developed on the surface of the liver.
   CT-vector: Colon carcinoma cell line infected with plasmid without LK68 gene,
   CT-LK68: Colon carcinoma cell line infected with plasmid containing LK68 gene,
   CT-LK8: Colon carcinoma cell line infected with plasmid containing LK8 gene
Fig. 5 is a set of photographs and graphs showing the histochemical morphology of liver, into which colon carcinoma cells were metastasized (a), the number of cells proved to be positive to PCNA staining, which was shown as percentage (b), and the number of cells proved to be positive to TUNEL, which was shown as percentage (c). To identify whether or not the number and the size of tumor nodule are significantly decreased, and whether or not apoptosis, but not the cell proliferation, is greatly increased in the area in liver metastasized with the LK68-transformed colon carcinoma cells, the major part of cancer developed area in the liver was examined with the histological or the immunohistochemical staining(at this time, H&E (Hematoxylin-eosin) staining was performed to observe the general metastasis, PCNA (proliferating cell nuclear antigen) staining and TUNEL (terminal deoxynucleotidyl transferase biotin-dUTP nick end labeling) were performed to observe the proliferation and apoptosis of cancer cells). From the total cell number, those cells proved to be positive to both PCNA staining and TUNEL were counted and the relative proportions were presented as % in graphs (the statistic significance was examined by student's t-test).
   CT-vector: Colon carcinoma cell line infected with plasmid without LK68 gene,
   CT-LK68: Colon carcinoma cell line infected with plasmid containing LK68 gene
Fig. 6 is a graph showing the survival rates of mice bearing liver metastasis in a liver dissemination model. Survival rates of both control (CT-vector) and colon carcinoma cell line (CT-LK68) carrying the LK68 gene were compared (The statistic significance was examined by log rank test of Kaplan-Meier survival curve).
   CT-vector: Colon carcinoma cell line infected with plasmid without LK68 gene,
   CT-LK68: Colon carcinoma cell line infected with plasmid containing LK68 gene
Fig. 7 is a graph showing the survival rates of mice bearing liver metastases in a peritoneal dissemination model. Survival rates of both control (CT-vector) and colon carcinoma cell line (CT-LK68) carrying the LK68 gene were compared (The statistic significance was examined by log rank test of Kaplan-Meier survival curve).
   CT-vector: Colon carcinoma cell line infected with plasmid without LK68 gene,
   CT-LK68: Colon carcinoma cell line infected with plasmid containing LK68 gene
Fig. 8 is a set of schematic diagrams and photographs showing the plasmid map of a vector containing LK68 or LK8, the result of agarose gel electrophoresis, and the nucleotide sequence encoding a FLAG. PCR was performed with a primer set that included a nucleotide sequence encoding FLAG, to introduce a binding site for FLAG to the end of carboxyl group of LK68 or LK8 protein.

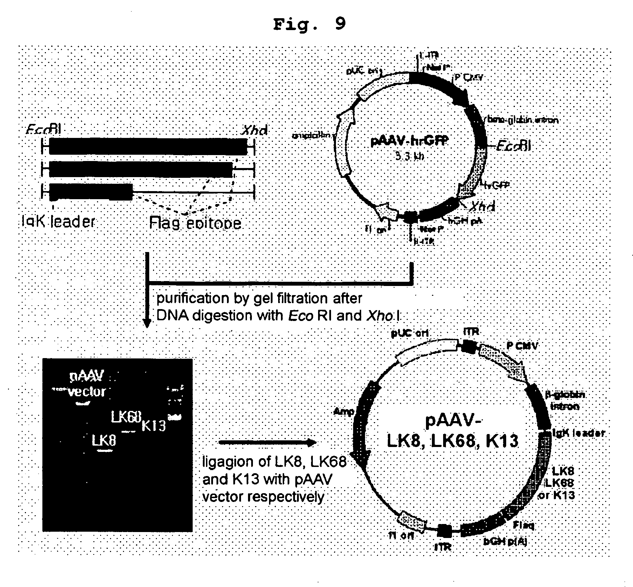

Fig. 9 is a set of schematic diagram and photograph showing the cloning of the amplified LK68 and LK8 DNA into the plasmid vector of adeno-associated virus and the result of agarose gel electrophoresis confirming the consequence of the cloning above.

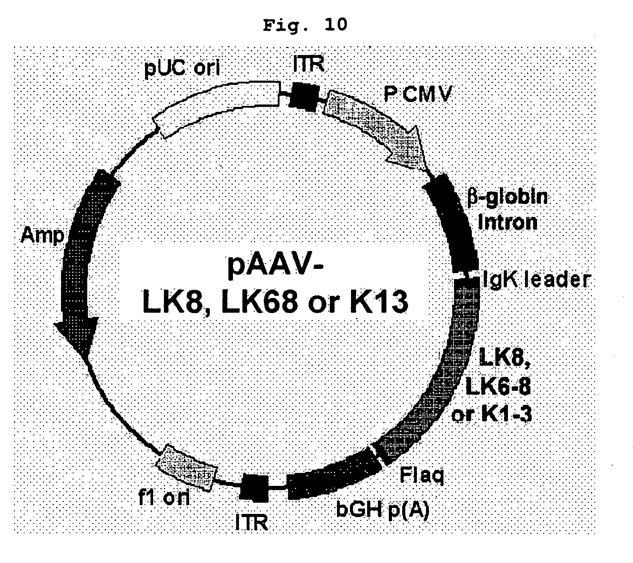

Fig. 10 is a plasmid map showing the location of each gene in the plasmid vector constructed by the cloning of Fig. 9.

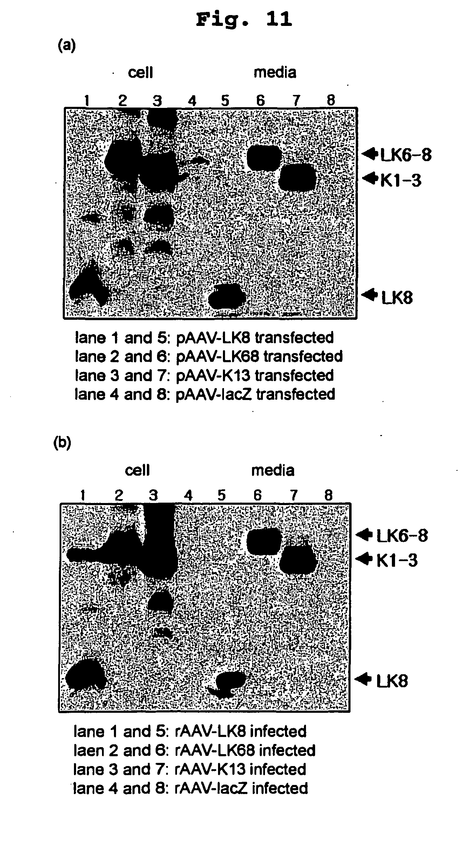

Fig. 11 is a set of photographs showing the results of Western blotting. The plasmid vector for the production of an adeno-associated virus gene carrier was transfected into HEK 293 cells *in vitro* to induce the expressions of LK68 and LK8 proteins, and the result was confirmed by Western blot analysis (a). The plasmid vector for the production of an adeno-associated virus gene carrier and a helper plasmid was co-transfected into HEK 293 cells to prepare a recombinant adeno-associated virus (rAAV), which was then used to infect HEK 293 cells *in vitro* to express LK68 and LK8 proteins, and the result was confirmed by Western blot analysis (b).

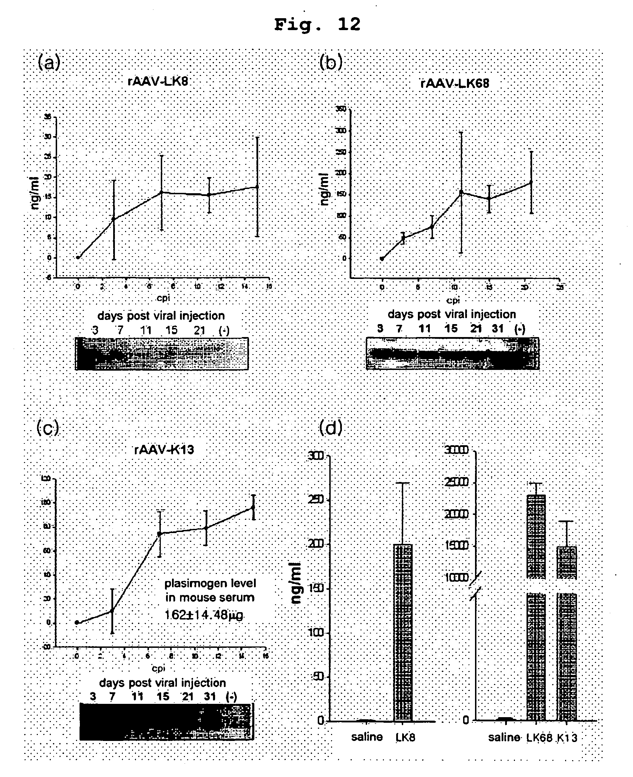

Fig. 12 is a set of photographs and graphs showing the expressions of genes in blood of mice. A recombinant adeno-associated virus containing LK8 gene (a), the 38th kringle of apolipoprotein(a), LK68 gene (b) or angiostatin (K13) gene (c) was injected into the muscle of hind limbs of Balb/c nude mice, then the expressions of those genes were examined. The plasmid containing LK68 or LK8 gene was delievered into C57BL/6 wild-type mice by a hydrodynamics-based transfection technique, then the expression of the above gene in blood was also investigated (d).

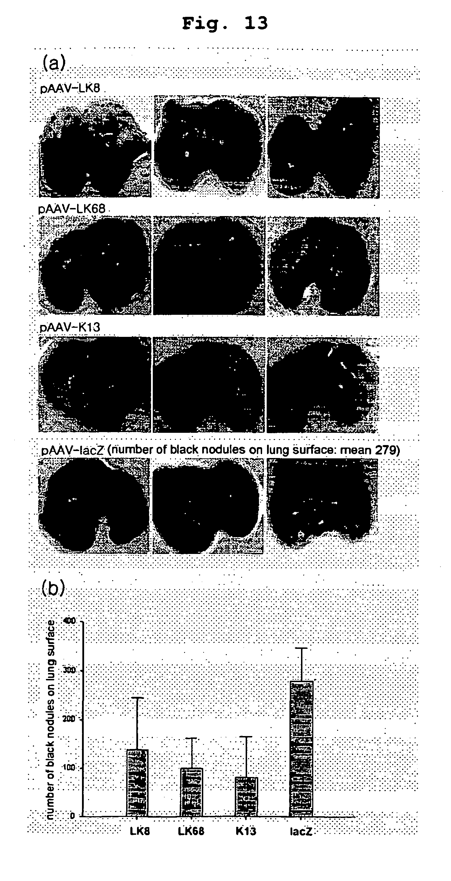

Fig. 13 is a set of a photograph (a) and a graph (b) showing that the metastasis of B16F10 tumor cells being transferred into the lung is inhibited in mice expressing LK68 or LK8 protein as a consequence of the transfer of the recombinant adeno-associated virus.

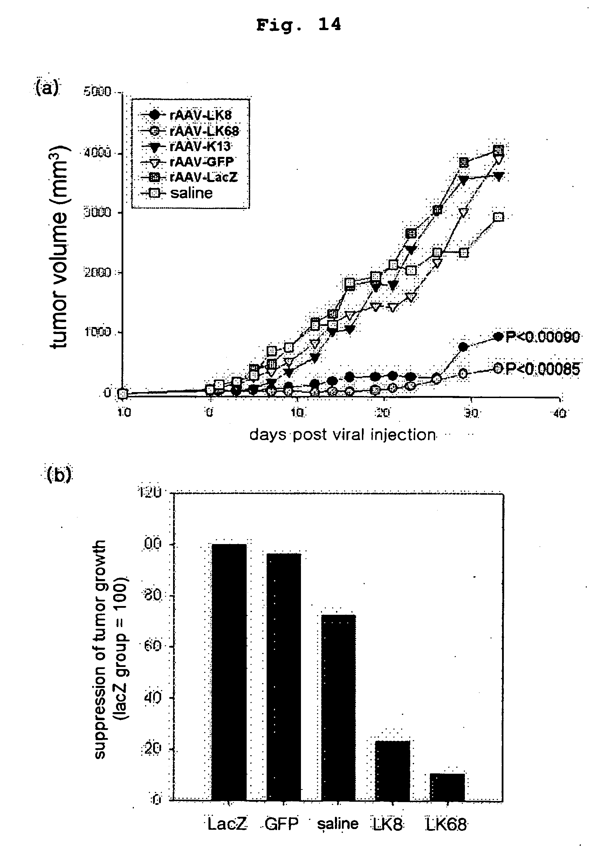

Fig. 14 is a set of graphs showing the growth curve (a) and the degree of the growth inhibition (b) of solid liver tumor. A solid liver tumor model was constructed by using Huh-7 hepatocellular carcinoma cells. And the growth and the growth inhibition of a solid liver tumor in the group treated with LK68 or LK8 gene carrier by intramuscular injection, and in the group not treated with it were compared with each other (at this time, the statistic significance was examined by Student's t-test).

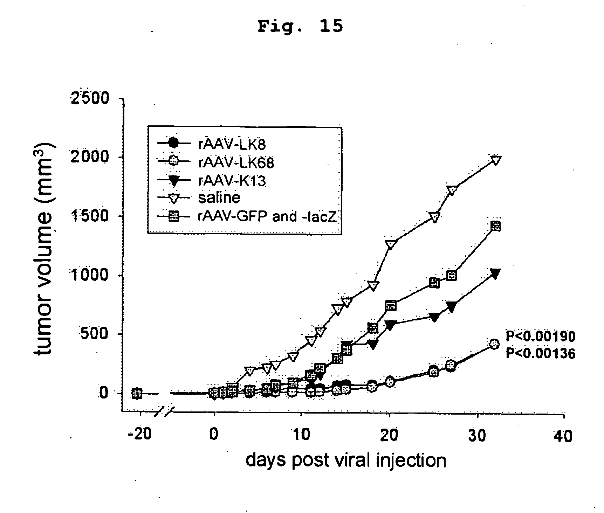

Fig. 15 is a graph showing the growth curve of solid liver tumor. A solid liver tumor model was constructed by using Hep3B hepatocellular carcinoma cells. The growth curves of the solid liver tumor in the group treated with LK68 and LK8 gene carrier by intramuscular injection, and in the group not treated with it were compared each other (at this time, the statistic significance was examined by Student's t-test).

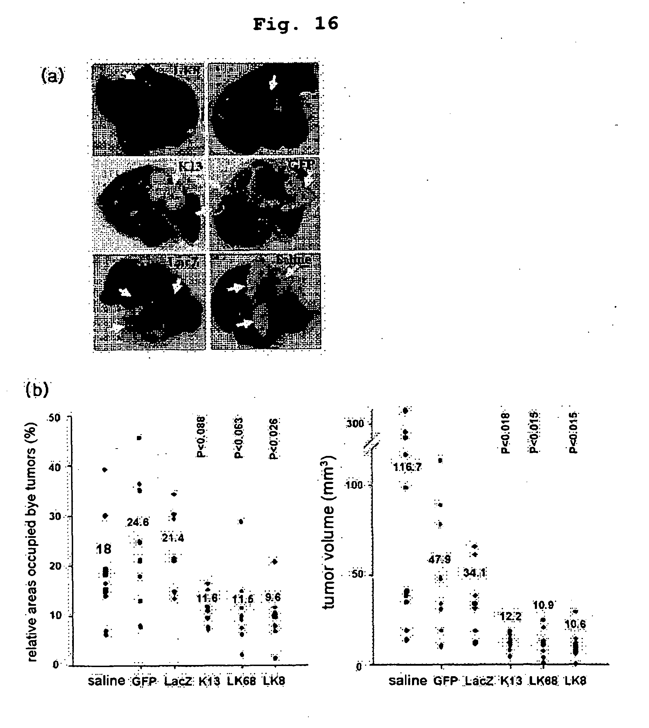

Fig. 16 is a set of photograph (a) and graph (b) showing the inhibitory effect of gene therapy on the growth of EL4 lymphoma cells. EL4 lymphoma cells were administered into the spleens of mice expressing LK68 and LK8 proteins as a consequence of the transfer of the recombinant adeno-associated virus (rAAV). The inhibition of metastasis to the liver from the spleen and the growth of the transferred tumor were investigated (the statistic significance was examined by Student's t-test and in order to help understand the figure, rAAV-GFP, -LacZ, -K13, -LK68 and -LK8 were represented by GFP, LacZ, K13 and LK8, respectively).

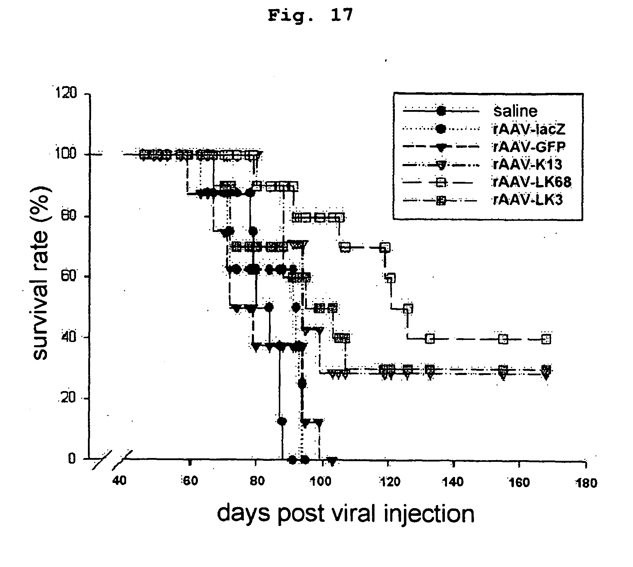

Fig. 17 is a graph showing the survival curves of the mice group treated with a recombinant virus containing LK68 and LK8 genes by intramuscular injection and the group not treated with it in a solid liver tumor model of huh-7 hepatocellular carcinoma cells (at this time, the statistic significance was examined by log rank test of Kaplan-Meier survival curve).

### Best Mode

### TERMINOLOGY

A gene carrier means a transport vehicle of a gene used to insert LK8 or LK68 gene into a treatment target. It includes promoter, enhancer, LK8 or LK68 gene and transcription terminator region, which are appropriate to be expressed in a target individual, and is selected from a group consisting of a vector containing a linear DNA fragment, a circular plasmid vector and a viral expression vector, a recombinant adenovirus, a recombinant retrovirus, a recombinant adeno-associated virus, a recombinant herpes simplex virus and a recombinant lentivirus. The promoter is one of organ or tissue specific promoters, and can include replication origin for the proliferation in target organs or tissues.

AAV stands for adeno-associated virus. It includes a recombinant adeno-associated virus expressing a foreign gene, but in the present invention, it means a wild-type virus unless there is a specific appointment. A recombinant adeno-associated virus (rAAV) means an adeno-associated virus that is able to express a foreign gene when the gene is inserted therein, and is a kind of a gene carrier that is utilized for gene therapy. The recombinant adeno-associated virus is also called as recombinant AAV vector. In the meantime, rAAV-LK8 means a LK8 expressing recombinant adeno-associated virus that is produced in cells transfected with pAAV-LK8, an expression vector of LK8 protein. Likewise, rAAV-LK68 means a recombinant adeno-associated virus expressing LK68 protein.

A recombinant adeno-associated viral expression vector (referred as 'rAAV expression vector' hereinafter) means, in a narrow sense, an expression vector containing a foreign gene that is designed to express the foreign gene in cells transfected with the recombinant adeno-associated virus. And in the broad sense, the rAAV expression vector includes AAV rep-cap gene expression vector, a helper plasmid or a helper virus, that is, it includes all the vectors that are able to construct a recombinant adeno-associated virus by transfecting cells. In the present invention, the rAAV expression vector means the last, unless there is a specific designation.

AAV *rep-cap* gene expression vector means an expression vector for genes that can encode, respectively, an enzyme (Rep) necessary for the copy of a genome originated from the recombinant adeno-associated virus expression vector and a capsid protein (Cap) available for the formation of adeno-associated virus particles, and can be inserted simultaneously with the recombinant adeno-associated virus expression vector, enabling the production of recombinant adeno-associated virus in cells. A helper virus is a virus that helps the formation of infectious adeno-associated virus particles that cannot be replicated by themselves, and is examplified by adenovirus, vaccinia virus and herpes simplex virus, etc. A helper plasmid, in the meantime, is a plasmid representing the functions of the helper virus. The mentioned AAV rep-cap gene expression vector and a helper virus can be embodied as one vector, and pDG (DKFZ, Germany) is the most represented example. Since all of (the AAV rep-cap gene expression vector and the helper virus or the helper plasmid can help rAAV expression vector, which can not form infectious adeno-associated virus particles by itself, to form infectious rAAV particles, the plasmid containing both AAV *rep-cap* gene and the minimal genes of the adenovirus necessary for the formation of infectious adeno-associated virus particles (ex: pDG) is called as a helper plasmid in the present invention. And the helper plasmid together with the helper virus is called 'helper vector'. Thus, the helper plasmid includes both the above AAV *rep-cap* gene expression vector and the expression vector for the minimal adenovirusgenes necessary for the formation of infectious adeno-associated virus particles, unless there is specific referring on it.

### DETAILED DESCRIPTION

In order to achieve the above objects, the present invention provides an anticancer or an anti-metastatic agent for gene therapy containing a gene carrier or cells harboring human apolipoprotein(a) kringle KIV9-KIV10-KV(LK68) or KV(LK8) gene as an effective ingredient.

The present invention also provides a method for the prevention or the treatment of a solid tumor, which includes a step of parenteral administration of a gene carrier or cells containing the LK68 or LK8 gene to an individual.

Hereinafter, the present invention is described in detail.

The present invention provides an anticancer or an anti-metastatic agent for gene therapy containing a gene carrier or cells harboring human apolipoprotein(a) kringle KIV9-KIV10-KV (LK68) or KV (LK8) gene as an effective ingredient.

The above LK68 gene comprises a nucleotide sequence represented by SEQ. ID. No. 1 preferably, but not always limited thereto. The gene carrier containing LK68 gene preferably includes linear DNA expressed in human or animals, plasmid vector, vector containing viral expression vector or recombinant retrovirus vector, recombinant adenovirus vector, recombinant adeno-associated virus vector, recombinant herpes simplex virus vector or recombinant virus vector containing recombinant lentivirus vector, but not always limited thereto. It is most preferred to select a gene carrier from a group consisting of pSecTag-LK68, pLXSN-LK68, rAAV-LK68 and pAAV-LK68. The cells containing LK68 gene are preferably selected from a group consisting of hematopoietic stem cells, dendritic cells, autologous tumor cells, and established tumor cells, but not always limited thereto, either.

The LK8 gene comprises a nucleotide sequence represented by SEQ. ID. No. 2 preferably, but not always limited thereto. The gene carrier containing LK8 gene preferably includes linear DNA expressed in human or animals, plasmid vector, vector containing viral expression vector or recombinant retrovirus vector, recombinant adenovirus vector, recombinant adeno-associated virus vector, recombinant herpes simplex virus vector or recombinant virus vector containing recombinant lentivirus vector, but not always limited thereto. It is most preferred to select a gene carrier from a group consisting of pSecTag-LK8, pLXSN-LK8, rAAV-LK8 and pAAV-LK8. The cells containing LK8 gene are preferably selected from a group consisting of hematopoietic stem cells, dendritic cells, autologous tumor cells, and established tumor cells, but not always limited thereto, either.

In the case that a therapeutic agent of the present invention is a vector containing LK68 or LK8 gene, 0.05 - 500 mg of dosage is preferred. And 0.1 ∼ 300 mg of dosage is more preferred. In the case that a therapeutic agent of the invention is a recombinant virus containing LK68 or LK8 gene, 10³ - 10¹² IU (10 - 10¹⁰ PFU) of dosage is preferred, and 10⁵ - 10¹⁰ IU of dosage is more preferred. In the case that a therapeutic agent of the invention is cells containing LK68 or LK8 gene, preferable number of cells to be included is 10³ - 10⁸, and more preferable number is 10⁴ - 10⁷.

The forementioned recombinant virus is preferred to be an adenovirus or an adeno-associated virus. The number of the virus required for the treatment is counted with virus particles containing a vector genome or the number of infectious virus. That is, effective number of infectious virus is under 1% of virus particles, which is given by the unit of IU (infection unit) or PFU (plaque forming unit).

The cells are utilized for the treatment using a recombinant retrovirus. For example, a recombinant retrovirus is utilized by the method of *ex* vivo transmission because it is inactivated by blood complement. Particularly, a recombinant retrovirus containing LK68 or LK8 is prepared first, then a target gene is delivered by the virus into human hematopoietic stem cells (CD34+), and lastly, the cells are administered to an individual for the treatment of a disease. At this time, the cells can be substituted with dendritic cells, autologous tumor cells, established tumor cells, etc. In numbers of preferred embodiments of the present invention, LK68 or LK8 gene was delivered into CT26 cell line, a kind of established tumor cells, by using a retrovirus vector and then the cells were administered to an individual, followed by further observation on the progress of a disease.

Such therapeutic agent of the present invention is preferably applied to the inhibition of cancer metastasis or the treatment of a primary tumor. And, a target cancer is preferably selected from a group consisting of colon carcinoma, liver cancer, lung cancer, breast cancer, brain tumor, prostatic carcinoma, skin cancer, stomach cancer, pancreas cancer, lymphoma, kidney cancer, ovarian cancer and metastatic tumor.

An agent for gene therapy of the present invention that contains a gene carrier or cells containing LK68 or LK8 gene as an effective ingredient can be administered parenterally and be used in general forms of pharmaceutical formulation.

The therapeutic agent of the present invention can be prepared for parenteral administration by mixing with generally used fillers, extenders, binders, wetting agents, disintegrating agents, diluents such as surfactant, or excipients. Formulations for parenteral administration are sterilized aqueous solutions, water-insoluble excipients, suspensions, emulsions, freeze-dried products, and suppositories. Water insoluble excipients and suspensions can contain, in addition to the active compound or compounds, propylene glycol, polyethylene glycol, vegetable oil like olive oil, injectable ester like ethylolate, etc. Suppositories can contain, in addition to the active compound or compounds, witepsol, macrogol, tween 61, cacao butter, laurin butter, glycerinated gelatin, etc. In order to enhance the pharmaceutical effect, calcium or vitamin D3 can be additionally included.

The dosage units can contain, for example, 1, 2, 3 or 4 individual doses or 1/2, 1/3 or 1/4 of an individual dose. An individual dose preferably contains the amount of active compound which is administered in one application and which usually corresponds to a whole, 1/2, 1/3 or 1/4 of a daily dose.

The therapeutic agent of the present invention can contain any of followings; a vector, a recombinant virus, and transfected cells. And the effective dosage of each case is respectively 0.05 - 12.5 mg/kg, 10⁷ - 10¹¹ particles/kg, and 10³ - 10⁶ cells/kg, and more preferably 0.1 - 10 mg/kg, 10⁸ - 10¹⁰ particles (10⁶ - 10⁸ IU) /kg, and 10² - 10⁵ cells/kg. And the administration times are 2 ∼ 3 times a day. The dosage is not always limited thereto and is determined according to the conditions of a patient and the severity of a disease.

The present inventors have confirmed that LK68 and LK8 proteins act as a very powerful anticancer agent both in vivo and ex vivo (Korean Patent Application No.: 2003-10797). Many of anticancer agents have to be administered continuously for a long term to keep the required level of a target protein, which causes problems of high production cost and resultant high unit prices of produced proteins, etc. LK68 and LK8 proteins are able to combine with many macromolecules (fibrinogen, fibrin, etc), which are major components of extracellular matrix, suggesting that they might be lost by a non-specific reaction with the macromolecules before they reached a target tumor.

To overcome such problems, gene therapy is one of alternatives. Precisely, a gene encoding a target protein, instead of the protein itself, is inserted into target cells *in vivo,* to express the protein directly in those transformed cells. For example, instead of inserting LK68 or LK8 protein, a kind of anticancer agent, a gene encoding the protein is inserted to express the protein in vivo, which can maximize the anticancer effect. Based on the idea, the present inventors obtained genes each having KIV9-KIV10-KV (LK68) structure and KV (LK8) structure only, among many kringle structures of human apolipoprotein(a), and then delivered those genes into tumor cells ex vivo, followed by insertion in a living body. The growth and the metastasis of the tumor cells were observed. As a result, it was confirmed that the growth and the metastasis of the tumor were successfully inhibited by the delivery of those genes.

The present inventors prepared a recombinant retrovirus having a gene coding LK68 or LK8 protein. Then, the virus was delivered into colon carcinoma cells ex vivo, and then transferred to a living body, followed by observation on the growth and the metastasis of the tumor cells. The present inventors also prepared a recombinant adeno-associated virus (rAAV), which is a therapeutic gene carrier, containing LK68 or LK8 gene. Then, the virus was inserted in a living body, followed by observation on the growth and the metastasis of the tumor cells in order to investigate the inhibiting effect of the system. The present inventors finally confirmed that the forementioned gene can be effectively used as an anticancer agent.

In the preferred embodiment of the present invention, the inventors prepared an about 1 kbps DNA containing genes encoding LK68 and LK8 proteins, which is corresponding to three kringle structures (KIV9, KIV10 and KV) of the end of apolipoprotein(a), from human liver cDNA library by RT-PCR. Compatibility of the obtained DNA was confirmed by an automatic sequencer, and then the DNA was named 'LK68 gene'. Only KV kringle region of the end of LK68 DNA was amplified with a selective primer, followed by cloning by the same method as used for the separation of LK68. The product was named 'LK8 gene'.

In order to construct a recombinant retrovirus, the prepared genes were cloned into retrovirus vector pLXSN (Clontech, USA). The vector containing LK68 gene was named 'pLXSN-LK68' and the vector containing LK8 gene was named 'pLXSN-LK8' (see Fig. 1). The recombinant vectors pLXSN-LK68 and pLXSN-LK8 were introduced into PT67 packaging cell line to produce a recombinant virus, by which LK68 or LK8 gene was delivered to a target mouse colon carcinoma cell line CT26. CT26 cell line stably expressing LK68 gene was named 'CT-LK68' and CT26 cell line stably expressing LK8 gene was named 'CT-LK8'. In the meantime, a cell line where only pLXSN vector was introduced without LK68 or LK8 was named 'CT-vector', which was a control.

The introduction of LK68 or LK8 gene did not affect directly the growth and apoptosis of a target cell (see Fig. 2). The anticancer effect of LK68 or LK8 protein expressed in CT-LK68 or CT-LK8 cell line was investigated by observing the growth and the metastasis of a solid tumor *in vivo* (see Fig. 3 to Fig. 7). As a result, it was confirmed that a gene therapy agent containing LK68 and LK8 inhibited the growth and the metastasis of a tumor excellently.

Based on the above results, the present inventors prepared a DNA by attaching a signal nucleotide sequence inducing extracellular secretion of immunoglobulin kappa (Igk) chain and FLAG antigen to the above LK68 and LK8 DNA. The DNA was introduced into an adeno-associated virus gene carrier delivery system to construct a recombinant adeno-associated virus vector containing LK68 or LK8 gene. The constructed vector was named 'pAAV-LK68' or 'pAAV-LK8'. And virus particles produced from the vector was named either 'rAAV-LK68' or 'rAAV-LK8' (see Fig. 8 - Fig.10). It was confirmed that the above recombinant gene carrier delivered LK68 or LK8 gene into cells or tissues successfully and the expressions of those genes thereby were also completed successfully (see Fig. 11). The expressed LK68 and LK8 proteins delivered by the recombinant virus were confirmed through animal tests to inhibit the metastasis of metastatic tumor B16F10 melanoma cells to lung by about 30 ∼ 60% (see Fig. 12 and Fig. 13), and the metastasis of metastatic tumor EL4 lymphoma cell to liver by 40 ∼ 90% (see Fig. 16). Therefore, it was confirmed that rAAV-LK68 or rAAV-LK8, recombinant adeno-associated viruses, may be used as an anticancer agent for gene therapy.

The present invention also provides a method for the prevention or the treatment of a solid tumor, which includes a step of parenteral administration of an anticancer or an anti-metastatic agent containing a gene carrier or cells having human apolipoprotein(a) kringle KIV9-KIV10-KV (LK68) or KV (LK8) gene as an effective ingredient to an individual. The expressed LK68 and LK8 proteins delivered by the recombinant virus were confirmed through animal tests to inhibit the tumor, growth of hepatocellular carcinoma Huh7 and Hep3B cells by about 80 ∼ 90% (see Fig. 14 and Fig. 15), and to increase the survival rate of the animal by 30 ∼ 40% in a solid liver tumor model of Huh-7 cells (see Fig. 17).

There is no limitation in administration methods of the above therapeutic agent, but it is preferred to select a method to introduce a gene carrier containing human apolipoprotein(a) kringle KIV9-KIV10-KV (LK68) or KV (LK8) gene into cells from a group consisting of chemical method, physical method, conjugation by using liposome, a method using receptor and virus, etc. The prevention or the treatment of a solid tumor in the present invention is preferably accomplished by the inhibition of the growth and the metastasis of the tumor. In another preferred embodiment of the present invention, the administration is preferably performed by the procedures of introducing human apolipoprotein(a) kringle KIV9-KIV10-KV (LK68) or KV (LK8) gene into cells selected from a group consisting of hematopoietic stem cells, dendritic cells, autologous tumor cells, and established tumor cells, and then administrating the cells *in vivo.*

The present inventors confirmed that the introduction of LK68 or LK8 gene showed anticancer effect by inhibiting the growth and the metastasis of a tumor in vivo (see Fig. 3 to Fig. 7), and a gene therapy agent containing LK68 or LK8 gene could effectively inhibit the growth of a tumor and the metastasis to other organs. And, LK68 and LK8 proteins expressed in cells after being delivered by the above gene carrier inhibited the metastasis of metastatic tumor B16F10 melanoma cells to lung by about 30 ∼ 60% (see Fig. 12 and Fig. 13). In addition, a recombinant adeno-associated virus gene carrier containing LK68 or LK8 gene was confirmed to inhibit metastasis of metastatic tumor EL4 lymphoma cell to liver by 40 - 90% (see Fig. 16).

### Mode for Invention

Practical and presently preferred embodiments of the present invention are illustrative as shown in the following Examples.

However, it will be appreciated that those skilled in the art, on consideration of this disclosure, may make modifications and improvements within the spirit and scope of the present invention.

### <Example 1> Construction of a colon carcinoma cell line expressing a recombinant protein LK68 or LK8

A gene encoding LK68 or LK8 protein was introduced into colon carcinoma cell line by using retrovirus. The construction procedure is precisely explained hereinafter.

### <1-1> Construction of recombinant vector expressing LK68 or LK8 protein

In order to obtain DNA fragments containing a gene encoding LK68 or LK8 protein (referred 'Lk68 or LK8 gene' hereinafter) separated from human liver, which was represented by SEQ. ID. No. 1 and SEQ. ID. No. 2, PCR was performed with a sense primer of LK68 gene represented by SEQ. ID. No. 3 or a sense primer of LK8 gene represented by SEQ. ID. No. 4 and a sense primer of a virus vector represented by SEQ. ID. No. 5, by following procedure; denaturation at 94°C for 5 minutes, then at 94°C for 30 seconds, annealing at 56°°C for 30 seconds, extension at 72°C for 1 minute, and the cycle was repeated 30 times, followed by additional reaction at 72°C for 5 minutes. Each sense primer was designed to include restriction enzyme *Sfi* I cleavage site, and an antisense primer was designed to harbor restriction enzyme *Xho* I cleavage site to make DNA ligation with a vector easy. PCR product was inserted into a vector, resulting in a recombinant vector. In order to induce the expression of a target protein (LK68 or LK8 protein) and the secretion of the target protein out of cells smoothly, ligation of the above PCR product with the digested area in pSecTag vector (Invitrogen, USA) by *Sfi* I and *Xho* I was performed, followed by the insertion to *Sfi* I restriction enzyme site of Igk leader sequence. As a result, each PCR product was set at upstreams of LK68 and LK8 genes, resulting in the construction of vectors 'pSecTag-LK68' and 'pSecTag-LK8'.

Then, in order to introduce LK68 or LK8 gene into retrovirus vector, PCR was performed with sense primer of virus vector represented by SEQ. ID. No. 6 and antisense primer of LK68 or LK8 represented by SEQ. ID. No. 5 by following procedure; denaturation at 94°C for 5 minutes, at 94°C for 30 seconds, annealing at 56°C for 30 seconds, extension at 72°C for 1 minute. After the cycle was repeated 30 times, reaction was additionally induced at 72°C for 5 more minutes. From the PCR, Igk-LK68 and Igk-LK8 DNA were selectively amplified from pSecTag-LK68 and pSecTag-LK8 vectors, respectively. At that time, the used primer harbored *Eco*R I and *Xho* I cleavage sites, to make DNA ligation with a vector easy. Igk-LK68 and Igk-LK8 DNA fragments amplified by PCR were digested with restriction enzymes *Eco*R I and *Xho* I. The DNA fragments were inserted into retrovirus vector pLXSN (Clontech, USA), which was digested in advance with the same restriction enzymes as the above, resulting in the construction of pLXSN-LK68 and pLXSN-LK8. The insertion of a target gene into a vector was confirmed by nucleotide sequence analysis and genetic map made by using a restriction enzyme (Fig. 1a).

### <1-2> Construction of colon carcinoma cell line harboring LK68 or LK8 gene

In order to prepare a recombinant retrovirus containing LK68 or LK8 gene, packaging cell line PT67 (Clontech, USA) was transfected with pLXSN-LK68 or pLXSN-LK8 vector constructed in the above example 1-1 by using a commercial liposome (Lipofectamin™, Life Technologies Inc., Rockville, MD, USA). The recombinant virus prepared from the transfected PT67 cell line was stored at -80°C, and if necessary for the transfection of colon carcinoma cell line CT26 (Korean Cell Line Bank), it was thawed. CT26 cell line transfected with the virus had resistance against antibiotics G418 (Conc.: 1 mg/ml) owing to a reporter gene included in pLXSN. Such resistance against G418 became a clue to secure a cell line expressing LK68 or LK8 protein stably. A cell line having LK68 gene inserted was named 'CT-LK68' and a cell line having LK8 gene inserted was named 'CT-LK8'. As a control group, a cell line containing only pLXSN without a gene coding the protein (LK68 or LK8) was named 'CT-vector', and a cell line that was not transfected with virus was named 'CT-mock'.

Western blot analysis and RT-PCR were performed to confirm whether or not LK68 and LK8 proteins were expressed in CT-LK68 and CT-LK8 cell lines (Fig. 1b and 1c). As a result, LK68 and LK8 proteins were successfully expressed in those cell lines.

### <Example 2> The in vitro growth of CT-LK68 and CT-LK8 cell lines and observation of expected apoptosis

In order to investigate the effect of the introduction of LK68 and LK8 genes in CT26 cells, the *in vitro* growth of CT-LK68 and CT-LK8 cell lines and expected apoptosis were observed.

Particularly, in order to observe the cell growth, CT-LK68, CT-LK8, CT-vector and CT-mock cell lines were distributed into each well of a 24 well tissue culture plate at the concentration of 2.5 ×10⁴ cells/well. Then, the cells were cultured in DMEM medium (Dulbecco's modified Eagles medium) supplemented with 10% FBS (fetal bovine serum), at 37°C in a 5% CO₂ incubator. Time-dependent cell growth was measured by trypan blue exclusion counting method (Freshney, R. I., 1994, Culture of Animal Cells. A manual of Basic Technique, 3rd Ed. Wiley-Liss. New York, USA) for 5 days. As a result, the equal cell growth was observed in both cell lines in which LK68 or LK8 gene was inserted and a control cell line (Fig. 2a; X-axis presents observation time and Y-axis presents the number of proliferated cells).

In order to confirm the expected apoptosis, about 1×10⁵ cells were stained with anexin-V and PI, followed by observation by FACS. CT26 cell line was treated with 10 µg/ml of taxol for 24 hours, then used as a positive control. While 83% apoptosis was observed in the positive control, which was CT26 cell line treated with 10 µg/ml of taxol, 0.89% apoptosis was observed in CT-mock cell line. 1.21% and 0.96% apoptosis were observed in CT-vector cell line and CT-LK68 cell line, respectively (Fig. 2b). The above results indicate that the expression of LK68 gene does not affect the growth of CT26 cell line and the expected apoptosis.

### <Example 3> Inhibition of a solid tumor growth by the insertion of LK68 gene

Whether or not LK68. protein expressed in colon carcinoma cells could inhibit the growth of a solid tumor was investigated in allogenic transplanted tumor model.

Particularly, 5×10⁵ CT-LK68 cells cultured in DMEM medium supplemented with 10% FBS were hypodermically injected in the center of dorsal of Balb/c mice (Charles River, Yokohama, Japan). Each group was composed of 8 - 10 mice. After transplantation of a tumor, the growth of the tumor was observed for one month. Control group mice were transplanted with CT-vector or CT-mock cells instead of CT-LK68 cells. The tumor size was measured every three or four days and the volume of the tumor was calculated by the formula of (length × width² × 0.52).

As a result, the tumor growth in CT-LK68 where LK68 gene was inserted was significantly inhibited, comparing to a control. Precisely, on the 33^{rd} day from the transplantation of the cell line, tumor growth rates were compared among CT-vector treated group, CT-mock treated group and CT-LK68 treated group, and as a result, the tumor growth was 80% inhibited in CT-LK68 treated group (Fig. 3a). Upon completing the observation, mice were sacrificed and tumors were separated from mice. The weights of those tumors were measured. The tumor in CT-LK68 treated group, in which LK68 gene was introduced, was lighter than that in CT-vector treated group which was similar to the results of measuring the volumes of those tumors (Fig. 3b and 3c).

### <Example 4> Inhibition of metastasis to liver from spleen by the introduction of LK68 gene

In order to investigate whether or not LK68 or LK8 protein which was expressed in colon carcinoma cells could inhibit metastasis of a solid tumor, an animal model was prepared by transplanting colon carcinoma cells into spleen of Balb/c mice, followed by observation on metastasis to liver.

Particularly, 5×10⁴ CT-LK68 cells were transplanted in spleen of Balb/c mice. On the 14^{th} day from the transplantation, liver was extracted from the mouse and the number of metastasized cancer colony was counted to determine the degree of metastasis. Fig. 4a is a photograph showing the most typical liver in which colon carcinoma cells were transferred. In the case of CT-vector treated group, abnormality was apparent in liver whose surface was covered with large or small cancer colonies. On the contrary, metastasis was significantly decreased in both CT-LK68 and CT-LK8 treated groups, and the shape of liver seemed to be like normal. The number of nodule formed on the surface of liver was also counted. As a result, the number of nodule was 47±30 per unit area in CT-LK68 treated group and 86±50 in CT-LK8 treated group. On the other hand, the nodule was 125±14 in CT-vector treated control group. The above results indicate that cell lines where LK68 or LK8 gene was inserted showed remarkably decreased number of nodules that were formed by metastasis, comparing to a control group (Fig. 4b).

The liver sample was fixed in formalin. Then, H&E staining was performed to observe general metastasis of colon carcinoma. PCNA staining was also performed to observe cancer cell growth, and immunohistochemical method of TUNEL staining was hired to investigate apoptosis (Fig. 5a, Junqueria et al., Basic Histology, Lange, 2003). The number of cells proved to be positive to each staining reagent was calculated as percentage to the total cell number in a certain area, in order to calculate growth index and apoptosis index of cancer cells.

The result of H&E staining suggested that general metastasis was decreased and the size of a tumor newly formed in the second location was very small in the host mouse liver in which CT-LK68 cells were transplanted, comparing to a control, a mouse transplanted with CT-vector. From the above results, it was confirmed that LK68 gene also inhibited the growing of micro metastasis into macro metastasis through angiogenesis.

The rates of PCNA stained cells to total cells were 64.5±3% and 64.3±2%, respectively in CT-LK68 treated group and in CT-vector treated group, showing the similar rates between two groups (Fig. 5b). However, the rates of TUNEL stained cells to total cells in CT-LK68 treated group and in CT-vector treated group were 5.36±1.35% and 0.47±0.14%, respectively, indicating that the expected apoptosis of cancer cells in CT-LK68 treated group increased significantly (Fig. 5c). The above results indicate that LK68 protein expressed in colon carcinoma cells does not affect the growth of cancer cells but effectively increases apoptosis of cancer cells. That is, the introduction of LK68 gene greatly contributes to the inhibition of the growth of colon carcinoma cells and the metastasis to liver.

Therefore, in consideration of all the results of H&E, PCNA and TUNEL, the metastasis inhibiting effect of LK68 gene insertion is believed to be resulted from the increase of expected apoptosis of cancer cells by the expression of LK68 protein.

### <Example 5> Increase of survival rate of a host by the introduction of LK68 gene in a liver metastasis model

An animal model, in which metastasis to liver of colon carcinoma cells transplanted in spleen was induced, prepared in the above example 4, was utilized to investigate the effect of the introduction of LK68 gene, confirmed earlier to inhibit the metastasis to liver, on survival rate of a host.

Particularly, 4×10⁵ CT-LK68 cells and CT-vector cells (a control) were respectively injected in spleens of mice, and then the condition and the viability of mice were examined every day. From the 23^{rd} day from the injection, mice transplanted with CT-vector began to die rapidly. 50% of the group survived 24 days but none survived over 28 days. In the meantime, 50% of the mice transplanted with CT-LK68 cells survived 36 days but none survived over 48 days (Fig. 6). The statistic significance of the survival rates of the two groups were examined by log rank test of Kaplan-Meier survival curve. From the results, it was confirmed that the insertion of LK68 gene contributed greatly to the increase of survival rate of a host by inhibiting metastasis.

### <Example 6> Increase of survival rate of a host by the introduction of LK68 gene in an abdominal cavity metastasis model

Colon carcinoma cells can be metastasized to abdominal cavity, in addition to the liver. The two ways of metastasis are the leading causes of recurrence after treatment and the death of a patient with colon carcinoma. Thus, the present inventors investigated whether or not the introduction of LK68 gene could increase viability of a host in an abdominal cavity metastasis model of colon carcinoma.

Particularly, 4×105 CT-LK68 cells and CT-vector cells (a control) were respectively injected in abdominal cavities of Balb/c mice utilized in the above example 3, followed by observation on the conditions and viabilities of those mice for 32 days. Death began to be reported rapidly in the group transplanted with CT-vector cells from the 14^{th} day from the transplantation. None survived over 21 days in the group. In the meantime, 50% of the group treated with CT-LK68 cells survived 23 days and none survived over 28 days (Fig. 7). The statistic significance of the difference in survival rates between the two groups was examined by log rank test of Kaplan-Meier survival curve. From the above results, it was confirmed that the introduction of LK68 gene contributed greatly to the increase of viability of a host by inhibiting metastasis of colon carcinoma cells to abdominal cavity.

### <Example 7> Construction of plasmid vectors pAAV-LK68 and pAAV-LK8 for the preparation of a recombinant adeno-associated virus gene carrier

The present inventors introduced LK68 and LK8 genes into adeno-associated virus vectors, then named them pAAV-LK68 and pAAV-LK8. The construction procedures of pAAV-LK68 and pAAV-LK8 plasmids are precisely explained hereinafter.

### <7-1> Amplification of LK68 or LK8 cDNA

In order to amplify LK68 DNA represented by SEQ. ID. No. 1, PCR was performed with primers represented by SEQ. ID. No. 7 and SEQ. ID. No. 8 by using human liver cell cDNA library (Clontech, Palo Alto, CA, USA) as a template with the same reaction condition and method as used in example 1-1. A base sequence encoding the end of amino group of LK68 was combined with signal nucleotide sequence of Igk, which was then cloned into pcDNA3.1(+) vector (Invitrogen, Carlsbad, CA, USA) to induce extracellular secretion of the protein. After cloning, nucleotide sequence of the DNA was analyzed by using a DNA sequencer (Applied Biosystems, CA, USA) to confirm if LK68 DNA was successfully inserted. The above vector was named pcDNA-LK68. PCR was performed with primers represented by SEQ. ID. No. 8 and SEQ. ID. No. 9 by using the above pcDNA-LK68 as a template to amplify LK8 DNA. LK8 DNA was cloned by the same method as used for the cloning of LK68 DNA, and the resultant vector was named pcDNA-LK8.

PCR was performed with primers represented by SEQ. ID. No. 10 and SEQ. ID. No. 11 by using pcDNA-LK68 and pcDNA-LK8 as templates to amplify LK68 and LK8 DNA. At that time, the primer represented by SEQ. ID. No. 10 was designed to bind with 5' end of signal nucleotide sequence of Igk which was linked to 5' end of LK68 and LK8 cDNA and include *Eco*R I recognition site at 5' end of the primer. The primer represented by SEQ. ID. No. 11 was designed to contain a nucleotide sequence encoding FLAG inside for the successful attachment of FLAG to 3' end of LK68 or LK8 cDNA. FLAG antigen marker was used to detect LK68 or LK8 DNA with ease.

100 ng of pcDNA-LK68 or pcDNA-LK8 vector, which was used as a template, 2 µl of each 25 mM primer, 5 µl of 10× PCR buffer, 4 µl of dNTP mixture (2.5 mM each) and 2.5 U of Ex-Taq polymerase were added in 0.2 ml of PCR tube. The final volume of the mixture was adjusted to 50 µl by using tertially distilled water, followed by PCR as follows; denaturation at 95°C for 5 minutes, at 95°C°C for 30 seconds, at 52°C°C for 30 seconds, at 72°C°C for 1 minute, and the cycle was repeated 30 times, then additional reaction was induced at 72°C°C for 3 minutes. Agarose gel electrophoresis was performed to confirm whether PCR was performed correctly (Fig. 8).

### <7-2> Construction of plasmid vectors pAAV-LK68 and pAAV-LK8

*Eco*R I and *Xho* I regions located before and after GFP gene of pAAV-hrGFP vector (Stratagene, La Jolla, CA, USA) were cut to eliminate GFP gene. Then, LK68 or LK8 cDNA was linked to the area.

Particularly, LK68 and LK8 DNA prepared by PCR were digested with restriction enzymes *Eco*R I and *Xho* I, and then DNA fragments were separated by gel extraction. The obtained DNA fragments were reacted with T4 DNA ligase at 16°C for 4 hours, leading to the transformation of competent cell line DH5α (ATCC) (Fig. 9). On the next day, DNA was extracted from the proliferated transformed DH5α cells. Digestion with *Eco*R I and *Xho* I was performed to confirm whether or not LK68 and LK8 DNA were successfully inserted into pAAV-hrGFP devoid of GFP gene (referred as 'pAAV' hereinafter). The vehicle plasmid vector where LK68 DNA was correctly inserted was named pAAV-LK68 and the vehicle plasmid vector where LK8 DNA was successfully inserted was named pAAV-LK8 (Fig. 10).

### <Example 8> Production and separation of a recombinant adeno-associated virus delivering LK68 or LK8 gene

In order to prepare a gene carrier delivering adeno-associated virus LK68 or LK8 gene, pAAV-LK68 or pAAV-LK8 DNA was mixed with a helper plasmid containing E2a, E4 and VA genes of adenovirus and the rep and cap genes of adeno-associated virus at the ratio of 1:5. The mixture was reacted with PEI (PolyPlus, Illkirch, France), which is a reagent for transformation, with the corresponding amount to the N/P ratio 5 at room temperature for 15 minutes. The resultant solution was dropped onto HEK293 cells (Microbix, Toronto, Ontario, Canada) where E1 gene of adenovirus was inserted and expressed stably, drop by drop, and then the cells were cultured at 37°C for 48 hours. The cultured cells were recovered, followed by centrifugation at 3,000 rpm for 10 minutes to separate the medium and cells. The cells were resuspended in a buffer composed of 0.15 M of NaCl and 50 mM of Tris-HCl (pH 8.5), followed by freezing and thawing three times, resulting in lysis of the cells. In order to eliminate DNA that was not covered with virus capsid, 50 U/ml of DNase was treated thereto for further reaction at 37°C for 30 minutes. Then, centrifugation was performed at 3,000 rpm for 20 minutes to separate supernatant. In order to isolate virus from the supernatant, iodixanol density gradient ultra-centrifugation was performed (Zolotukhin, S. et al., Gene Therapy, 6: 973-985, 1999). The resultant virus prepared by the above method was named each rAAV-LK68 or rAAV-LK8.

### <Example 9> Expression of LK68 or LK8 gene delivered by a recombinant adeno-associated virus

In order to confirm whether or not LK68 or LK8 gene delivered by vehicle plasmid vectors pAAV-LK68 and pAAV-LK8 prepared in the above example 8 was expressed successfully, the plasmid vectors were inserted in HEK 293 cells *in vitro,* then expression and extracellular secretion of LK68 or LK8 gene were investigated in vivo and *in vitro* as well.

### <9-1> Expression of LK68 or LK8 gene by in vitro transduction and an adeno-associated virus gene carrier

HEK 293 cells that were grown 70-80% to confluency in 6-well plate were transfected with 3 mg of pAAV-LK68 or pAAV-LK8 plasmid vector by using PEI reagent (PolyPlus, Illkirch, France), followed by further culture at 37°C for 24 hours.

In the meantime, HEK 293 cells grown 70-80% to confluency in 6-well plate without FBS were transfected with rAAV-LK68 or rAAV-LK8, which was prepared in the above example 8, with the inoculum size of 5 multiplicity of infection per cell. One hour later, the medium was replaced with a fresh one, followed by additional culture at 37°C for 24 hours.

The above cells cultured differently were recovered, followed by centrifugation at 3,000 rpm for 5 minutes to separate cells and medium. The cells were resuspended in 1× SDS-PAGE buffer (50 mM Tris (pH 6.8), 2% SDS (sodium dodecyl sulfate), 100 mM DTT (dithiothreitol), 0.1% BPB (bromophenol blue), 10% glycerol), and then boiled at 100°C for 5 minutes. Centrifugation was performed at 10,000 rpm for 2 minutes to separate supernatant. The medium was 10 fold concentrated by using a concentrator (Centricon^{®} YM-10; Amicon, Beverly, MA, USA), resulting in preparation of a sample by the same method as used above.

Electrophoresis was performed in 15% SDS-PAGE at 20 mA for 2 hours by using an electrophoresis kit. Electrophoresis was performed again in tris-glycine buffer (39 mM glycin, 48 mM tris, 0.037% SDS, 20% methanol) at 300 mA for 90 minutes by using transfer unit, leading to the movement to PVDF membrane (polyvinylidene fluoride membrane). Overnight blocking was carried out at room temperature by using 5% skim milk/1× TBST buffer (10 mM Tris, 150 mM NaCl, 0.1% Tween-20). Mouse anti-FLAG monoclonal antibody (Sigma, St. Louis, MO, USA), as the primary antibody, was diluted in 1× TBST buffer at the ratio of 1:3,000, which was left at room temperature for about one hour for reaction. Then, the reaction solution was washed with TBST buffer 6 times at five minutes interval. Mouse anti-HRP (KPL, Gaithersburg, MD, USA), as the secondary antibody, was also diluted in 1× TBST buffer at the ratio of 1:5,000, leading to reaction for 30 minutes. The solution was washed with TBST buffer 6 times at five minutes interval. The expression of LK68 or LK8 protein was confirmed by using a chemiluminescent kit (ECL™ Chemiluminescent Western Blotting Detection Reagents kit; Amersham, Buckinghamshire, UK) (Fig. 11a and 11b).

### <9-2> Expression of LK68 gene in Balb/c nude mice

Apolipoprotein(a) is not an assets of a mouse. Thus, the present inventors expected that when LK68 gene prepared by the inventors was inserted in a wild-type mouse, the LK68 protein would be destroyed by the immune response occurring in the mouse by antibody formation against the protein.

Adeno-associated virus gene carriers, rAAV-LK68 and rAAV-LK8, prepared and separated in the above example 8, were injected in three different spots of hind leg muscle of Balb/c nude mouse utilized in the above example 3 by the concentration of 1×10⁹ IU (infection unit, representing the unit of infectious virus). Only saline was injected for the negative control and rAAV-K13, a recombinant adeno-associated virus expressing angiostatin (K13) was injected for the positive control. Blood was taken from ocular venous plexus at three days interval, which was continued until total 100.gl of blood was taken. Then, protease inhibitor cocktail was treated. The blood sample was left at room temperature for about one hour, followed by centrifugation at 12,000 rpm for 10 minutes, resulting in the separation of erythrocytes and plasma. Some of the separated plasma was analyzed by ELISA to measure the amounts of LK68, LK8 and K13 genes expressed in blood. Western blot analysis was also performed, like in example 9-1, to investigate the expressions of those proteins. ELISA took advantage of two different antibodies having different serotypes against kringles (LK8 protein) of the end of carboxylic acid among three kringles of LK68 protein. One of them was rabbit anti-LK8 antibody and the other was mouse anti-LK8 antibody. Rabbit anti-LK8 antibody was mixed with coating buffer (10 mM PBS, pH 7.2) at the concentration of 0.25 mg/section in a plate (F8 MAXISORP LOOSE; Nunc, Rosklide, Denmark) designed to be bound well to immunoglobulin (Ig), which was left at 37°C for one hour and then washed with a washing buffer (1× PBS, 0.1% Tween 20) three times. 200 ml of washing buffer supplemented with 1% BSA (bovine serum albumin) was added, followed by blocking at room temperature for two hours. The obtained plasma was diluted at different concentrations, which were reacted at 37°C for one hour, and then washed with a washing buffer three times. Mouse anti-LK8 antibody was diluted at the ratio of 1:1,000, which was left for reaction at 37°C for one hour. Finally, antimouse HRP (KPL, Gaithersburg, MD, USA) was diluted at the ratio of 1:20,000, and then washed with a washing buffer three times. TMB enzyme substrate (KPL, Gaithersburg, MD, USA) was treated thereto. Color development was investigated by a photometer set at 450 nm to measure the expression of LK68 in blood, and the result was compared with that of LK68 protein comparative group quantified already. The expression of LK8 gene in blood was also measured by comparing with that of LK8 protein comparative group quantified already. The expression of K13 was also investigated by the same method as used above.

As a result, the expression of LK68 gene delivered by a virus carrier increased gradually and reached 150 ∼ 200 ng/ml 2 weeks after the delivery. The expression of LK8 gene was also increased gradually like LK68 gene, which was 20 ∼ 30 ng/ml, but the total amount of expression was less than that of LK68 (Fig. 12a, 12b and 12c).

### <9-3> Expression of LK68 or LK8 gene inserted by hydrodynamic technique

1.8 ml of 0.25% saline including plasmid each containing 25 mg of LK68 or LK8 gene, was injected in the tail vein of C57BL/6 wild type mice (Charles River, Yokohama, Japan) within 7 seconds. For a negative control, only saline without any plasmid was injected, and rAAV-K13 was injected for a positive control. After one week from the injection, blood was taken from ocular venous plexus to measure the expression of LK68 or LK8 gene in blood. As a result, the expression amount of LK68 protein was 20 ~ 25 mg/ml, and the expression amount of LK8 protein was 200 ∼ 250 ng/ml (Fig. 12d). These results were compared with the expressions of the proteins led by the adeno-associated virus carrier. As a result, the expression of LK68 was 100 fold higher and the expression of LK8 was 10 fold higher, indicating that the above method was more effective. That is, hydrodynamic injection induces higher expression of a gene than a virus carrier does because it enables fast DNA injection (within seconds) inducing high expression of a target gene shortly after the injection *in vivo* (Liu, F. et al., Gene Therapy, 6: 1258-1266, 1999).

### <Example 10> Inhibition of the growth of a metastatic tumor by the insertion of LK68 or LK8 gene

The present inventors confirmed that a protein expressed by LK68 or LK8 gene inhibited the growth and the metastasis of a metastatic tumor *in vivo.*

Particularly, 1×10⁹ IU of adeno-associated virus LK68 gene carrier (rAAV-LK68) or LK8 gene carrier (rAAV-LK8), prepared in the above example 8, was injected in three different spots of muscle of hind legs of Balb/c nude mice utilized in the above example 3. Then, blood was taken from ocular venous plexus at three days interval to investigate the expression of LK68 or LK8 gene in blood. At that time, rAAV-lacz, in which pAAV-lacZ (Stratagene, USA) expressing β-galactosidase was inserted, was used for a negative control, and rAAV-K13 was used for a positive control.

After two weeks from the injection of recombinant adeno-associated virus LK68 or LK8 gene carrier, 2×10⁵ B16F10 melanoma cells (ATCC, Manassas, VA, USA) were injected in the tail vein. B16F10 melanoma cells are the cells largely transferring to the lung, and in general, small black process-like tumor cells are formed on the surface of the lung from 2 weeks after the injection (Fidler, I.J. et al., J. Natl. Cancer Inst., 57; 1199-1202, 1976). After 2 weeks from the injection of B16F10 melanoma cells, the lung of a mouse was taken to count the number of black processes formed on the surface of the lung. As a result, it was confirmed that metastasis was about 30 ∼ 60% inhibited (Fig. 13).

### <Example 11> Inhibition of the growth and the metastasis of a tumor by the insertion of LK68 or LK8 gene in a liver tumor model

The present inventors investigated whether or not a protein expressed by LK68 or LK8 gene, like angiostatin (K13), could inhibit the growth and the metastasis of a liver tumor *in vivo.*

### <11-1> Inhibition of the growth of a liver tumor by the insertion of LK68 or LK8 gene carrier in a liver tumor model using Huh-7 cells (Human hepatocellular carcinoma cells)

Huh-7 cells (JCRB, Tokyo, Japan) were washed with PBS twice, and then 1×10⁷ cells were injected hypodermically in the area of the right flank of Balb/c nude mice (Charles River, Wilmington, MA, USA) to induce a solid liver cancer. From the 10^{th} day of injection, a solid liver cancer began to be growing, showing over 80% development rate. The volume of the tumor was measured everyday by the formula [length×width²×0.52]. When the tumor was grown up to 50 mm³, 1×10⁹ IU of adeno-associated virus LK68 gene carrier (rAAV-LK68) or LK8 gene carrier (rAAV-LK8), prepared in the above example 8, was injected in three different spots of muscle of hind legs of Balb/c nude mice. After 2 weeks from the injection of LK8 or LK68 gene carrier, blood was taken from ocular venous plexus to investigate the expression of LK68 or LK8 gene in blood, during which the growth of the tumor was checked everyday. Only saline was injected for a negative control, and rAAV-K13 was injected for a positive control. In order to examine infection efficiency, rAAV-GFP, in which pAAV-hrGFP (Stratagene, USA) was inserted, and rAAV-lacZ were used. As a result, the expression amount of LK68 was 50 ~ 150 ng/ml, and the expression amount of LK8 was 30 ~ 50 ng/ml. In the meantime, the expression amount of K13 was 50 ∼ 100 ng/ml. Both LK68 and LK8 inhibited the solid liver tumor growth by 80 ∼ 90% (Fig. 14).

### <11-2> Inhibition of the growth of a liver tumor by the insertion of LK68 or LK8 gene carrier in a liver tumor model using Hep3B hepatoma cells

Another solid liver tumor was induced using Hep3B hepatocellular carcinoma cells (ATCC, Manassas, VA, USA) by the same method as used in example 11-1. From the 21^{st} day, the solid tumor began to be growing, showing about 30% of tumorization efficiency. When the tumor was grown up to 50 mm³, LK68 or LK8 gene carrier was injected into muscle, and the growth of the solid tumor in liver induced by Hep3B cells and the expression of LK gene were investigated. As a result, the expression amount of LK8 or LK68 was similar to the result of example 11-1, and the growth of the solid tumor was inhibited roughly by 80% (Fig. 15a and 15b).

### <11-3> LK68 or LK8 activity in a metastatic liver tumor model using EL4 human lymphoma cells

Spleens of male Balb/c nude mice, in which LK68 was expressed by 50 ∼ 100 ng/ml and LK8 was expressed by 30 ∼ 50 ng/ml by the intramuscular injection of LK68 or LK8 gene carrier, were opened, and 50 µ1 of 2×10⁵ EL4 lymphoma cells (ATCC, Manassas, VA, USA) were slowly injected therein. Only saline was injected for a negative control and rAAV-K13 was injected for a positive control. In order to investigate the infection efficiency, rAAV-GFP and rAAV-lacZ were used. The mice were taken care not to be exposed on ether steam too much and 5 minutes were allowed the mice to make the inserted cancer cells move to the hepatic portal vein. Then, the spleens were sutured. About 2 weeks later, livers were taken to investigate the size and the number of transferred cancer cells and also tumor area was investigated by an analyzing program (SigmaScan^{®} Pro 5.0, Systat Software, Point Richmond, CA, USA). As a result, the tumor size was 68 ∼ 90% smaller by rAAV-LK68 administration, and 70 ~ 91% smaller by rAAV-LK8 administration, comparing to a control group. In the meantime, the area of the transferred tumor in liver was inhibited 37 ∼ 54% by rAAV-LK68 administration and 47 ∼ 61% by rAAV-LK8 administration (Fig. 16a and 16b).

### <11-4> Increase of survival rate by LK68 or LK8 in a solid liver tumor model using Huh-7 cells

The above results indicate that LK68 or LK8 can effectively inhibit the growth and the metastasis of a cancer. In order to confirm how much the treatment effect of LK68 or LK8 increases the viability of a host, the growth of liver cancer was observed until the host died of it in a solid liver tumor animal model using Huh-7 cells used in example 11-1. As a result, in comparative groups where LK68 and LK8 gene carriers were not injected, animals began to be killed 60 days after the injection of liver cancer cells and none survived 100 days. On the contrary, in the experimental group injected with LK68 gene carrier, animals began to be killed 80 days after the injection of liver cancer cells and even after 140 days from the injection, survival rate was 40%. In the other experimental group treated with LK8 gene carrier, animals began to be killed 68 days after the injection of liver cancer cells and after 130 days from the injection, survival rate reached 30%. The statistic significance of the difference in survival rates between a gene carrier treated group and a gene carrier non-treated group was examined by log rank test of Kaplan-Meier survival curve. From the above results, it was confirmed that the introduction of LK68 or LK8 gene by the delivery on a gene carrier greatly contributed to the inhibition of the growth of a solid tumor, leading to the increase of viability of a host (Fig. 17).

### Industrial Applicability

As explained hereinbefore, the present inventors prepared a recombinant cell line (CT-LK68 or CT-LK8) harboring LK68 or LK8 gene by gene manipulation technique *in vitro,* and then confirmed that the inserted LK68 or LK8 gene inhibited the growth and the metastasis of a tumor *in vivo* (*ex vivo* gene therapy). In the meantime, the present inventors prepared an adeno-associated virus gene carrier in which LK68 or LK8 gene was inserted, and injected the carrier directly in vivo, followed by confirmation that the inserted LK68 or LK8 gene inhibited the growth and the metastasis of a tumor effectively (in vivo gene therapy). In conclusion, LK68 or LK8 gene of the present invention inhibits the growth of a metastatic tumor, and can be delivered more efficiently by a gene carrier, so that they can be effectively used for the prevention of various cancer-related diseases and for gene therapy as well as for the development of an anticancer agent.

### Sequence List Text

The SEQ. ID. No. 1 is a gene coding LK68 protein that is originated from human liver apolipoprotein(a);
The SEQ. ID. No. 2 is a gene coding LK8 protein that is originated from human liver apolipoprotein(a),;
The SEQ. ID. No. 3 and No. 4 are sense primers for the amplification of genes each coding the above LK68 protein and the LK8 protein;
The SEQ. ID. No. 5 is an antisense primer originated from a virus vector for the amplification of a gene coding the above LK68 or LK8 protein;
The SEQ. ID. No. 6 is a sense primer originated from a virus vector for the introduction of a gene coding the above LK68 or LK8 protein;
The SEQ. ID. No. 7 and No. 8 are sense primer and antisense primer, respectively, for the amplification of LK68 cDNA;
The SEQ. ID. No. 9 is a sense primer for the amplification of LK8 cDNA, along with the SEQ. ID. No. 8;
The SEQ. ID. No. 10 is a sense primer for the amplification of DNA fragment of LK68 or LK8; and
The SEQ. ID. No. 11 is an antisense primer for the amplification of DNA fragment of LK68 or LK8.
The SEQ. ID. No. 1 ∼ No. 11 are presented in the below sequence list.

## Claims

1. An anticancer or an anti-metastatic agent for gene therapy containing
(a) a retroviral vector or retrovirus harboring the human apolipoprotein(a) kringle KIV9-KIV10-KV (LK68) or KV (LK8) gene as an effective ingredient;
(b) an adeno-associated viral vector or adeno-associated virus harboring the human apolipoprotein(a) kringle KIV9-KIV10-KV (LK68) or KV (LK8) gene as an effective ingredient;
(c) a plasmid DNA harboring the human apolipoprotein(a) kringle KIV9-KIV10-KV (LK68) or KV (LK8) gene as an effective ingredient;
(d) an adenoviral vector or adenovirus harboring the human apolipoprotein(a) kringle KIV9-KIV10-KV (LK68) or KV (LK8) gene as an effective ingredient; or
(e) cells harboring the retroviral vector or retrovirus of (a), the adeno-associated viral vector or adeno-associated virus of (b), the plasmid DNA of (c) or the adenoviral vector or adenovirus of (d).

2. The agent according to claim 1, wherein the LK68 gene comprises the nucleotide sequence represented by SEQ. ID. No:1.

3. The agent according to claim 1, wherein the LK8 gene comprises the nucleotide sequence represented by SEQ. ID. No:2.

4. The agent according to claim 1, wherein the retroviral vector is pLXSN-LK8 or pLXSN-LK68 illustrated in Fig. 1.

5. The agent according to claim 1, wherein the adeno-associated vector is pAAV-LK8 or pAAV-LK68 illustrated in Fig. 10.

6. The agent according to claim 1, wherein the plasmid DNA is selected from the group consisting of pSecTag-LK8, pSecTag-LK68, pcDNA-LK8 and pcDNA-LK68.

7. The agent according to claim 1, wherein the cells are selected from the group consisting of hematopoietic stem cells, dendritic cells, autologous tumor cells and established tumor cells.

8. The agent according to claim 1, wherein the retroviral vector of (a), the adeno-associated viral vector of (b), the plasmid DNA of (c) or the adenoviral vector of (d) is included by 0.05 ∼ 500 mg.

9. The agent according to claim 1, wherein the retrovirus of (a), the adeno-associated virus of (b) or the adenovirus of (d) is included by 10³-10¹² IU.

10. The agent according to claim 1, wherein the cells are included by 10³-10⁸ e.a.

11. The agent according to claim 1, wherein the cancer is selected from the group consisting of colon carcinoma, liver cancer, lung cancer, breast cancer, brain tumor, prostatic carcinoma, skin cancer, stomach cancer, pancreas cancer, lymphoma, kidney cancer, ovarian cancer and metastatic tumor.

12. The agent according to claim 11, wherein the cancer is selected from the group consisting of colon carcinoma, liver cancer, lymphoma and metastatic tumor.

13. Use of the agent for gene therapy of any one of claims 1 to 12 in the preparation of a medicament for the prevention or the treatment of a solid tumor, wherein the medicament is to be administered parenterally.

14. Use of the agent for gene therapy of any one of claims 1 to 12 in the preparation of a medicament for the inhibition of the growth and the metastasis of a solid tumor.

15. The use of claim 13 or 14, wherein the medicament is administrated by hypodermical injection, hydrodynamic injection, intratumoral injection or an intramuscular injection.

## Patentansprüche

1. Antikrebs- oder anti-metastatisches Mittel zur Gentherapie enthaltend
(a) einen retroviralen Vektor oder ein Retrovirus mit dem menschlichen Apolipoprotein(a) Kringle KIV9-KIV10-KV (LK68)- oder KV (LK8)-Gen als Wirkstoff;
(b) einen Adeno-assoziierten viralen Vektor oder ein Adeno-assoziiertes Virus mit dem menschlichen Apolipoprotein(a) Kringle KIV9-KIV10-KV (LK68)- oder KV (LK8)-Gen als Wirkstoff;
(c) eine Plasmid-DNA mit dem menschlichen Apolipoprotein(a) Kringle K1V9-K1V1O-KV (LK68)- oder KV (LK8)-Gen als Wirkstoff;
(d) einen adenoviralen Vektor oder ein Adenovirus mit dem menschlichen Apolipoprotein(a) Kringle KIV9-KIV10-KV (LK68)- oder KV (LK8)-Gen als Wirkstoff; oder
(e) Zellen, welche den retroviralen Vektor oder das Retrovirus von (a), den Adeno-assoziierten viralen Vektor oder das Adeno-assozüerte Virus von (b), die Plasmid-DNA von (c), oder den adenoviralen Vektor oder das Adenovirus von (d) beherbergen.

2. Mittel nach Anspruch 1, wobei das LK68-Gen die Nukleotidsequenz wie in SEQ. ID. No: 1 dargestellt umfasst.

3. Mittel nach Anspruch 1, wobei das LK68-Gen die Nukleotidsequenz wie in SEQ. ID. No: 2 dargestellt umfasst.

4. Mittel nach Anspruch 1, wobei der retrovirale Vektor pLXSN-LK8 oder pLXSN-LK68 wie in Fig. 1 dargestellt ist.

5. Mittel nach Anspruch 1, wobei der Adeno-assozüerte Vektor pAAV-LK8 oder pAAV-LK68 wie in Fig. 10 dargestellt ist.

6. Mittel nach Anspruch 1, wobei die Plasmid-DNA ausgewählt ist aus der Gruppe bestehend aus pSecTag-LK8, pSecTag-LK68, pcDNA-LK8 und pcDNA-LK68.

7. Mittel nach Anspruch 1, wobei die Zellen ausgewählt sind aus der Gruppe bestehend aus hämatopoietischen Stammzellen, dendritischen Zellen, autologen Tumorzellen und etablierten Tumorzellen.

8. Mittel nach Anspruch 1, wobei der retrovirale Vektor von (a), der Adeno-assozüerte virale Vektor von (b), die Plasmid-DNA von (c) oder der adenovirale Vektor von (d) in einem Bereich von 0,05 ∼ 500 mg enthalten ist.

9. Mittel nach Anspruch 1, wobei das Retrovirus von (a), das Adeno-assozüerte Virus von (b), oder das Adenovirus von (d) in einem Bereich von 10³-10¹² IU enthalten ist.

10. Mittel nach Anspruch 1, wobei die Zellen in einem Bereich von 10³-10⁸ e.a. enthalten sind.

11. Mittel nach Anspruch 1, wobei der Krebs ausgewählt ist aus der Gruppe bestehend aus Darmkarzinom, Leberkrebs, Lungenkrebs, Brustkrebs, Gehirntumor, Prostatakarzinom, Hautkrebs, Magenkrebs, Bauchspeicheldrüsenkrebs, Lymphom, Nierenkrebs, Eierstockkrebs und metastatischem Tumor.

12. Mittel nach Anspruch 11, wobei der Krebs ausgewählt ist aus der Gruppe bestehend aus Darmkarzinom, Leberkrebs, Lymphom, und metastatischem Tumor.

13. Verwendung des Mittels zur Gentherapie nach einem der Ansprüche 1 bis 12 zur Herstellung eines Medikaments für die Vorbeugung oder Behandlung eines soliden Tumors, wobei das Medikament parenteral zu verabreichen ist.

14. Verwendung des Mittels zur Gentherapie nach einem der Ansprüche 1 bis 12 zur Herstellung eines Medikaments für die Hemmung des Wachstums und der Metastasierung eines soliden Tumors.

15. Verwendung nach Anspruch 13 oder 14, wobei das Medikament durch subkutane Injektion, hydrodynamische Injektion, intratumorale Injektion oder intramuskuläre Injektion verabreicht wird.

## Revendications

1. Agent anticancéreux ou antimétastasique pour thérapie génique contenant
(a) un vecteur rétroviral ou un rétrovirus hébergeant, à titre d'ingrédient efficace, le gène KV (LK8) ou KIV9-KIV10-KV (LK68) de kringle d'apolipoprotéine(a) humain ;
(b) un vecteur viral adéno-associé ou un virus adéno-associé hébergeant, à titre d'ingrédient efficace, le gène KV (LK8) ou KIV9-KIV10-KV (LK68) de kringle d'apolipoprotéine(a) humain ;
(c) un ADN plasmidique hébergeant, à titre d'ingrédient efficace, le gène KV (LK8) ou KIV9-KIV10-KV (LK68) de kringle d'apolipoprotéine(a) humain ;
(d) un vecteur adénoviral ou un adénovirus hébergeant, à titre d'ingrédient efficace, le gène KV (LK8) ou KIV9-KIV10-KV (LK68) de kringle d'apolipoprotéine(a) humain ; ou
(e) des cellules hébergeant le vecteur rétroviral ou le rétrovirus de (a), le vecteur viral adéno-associé ou le virus adéno-associé de (b), l'ADN plasmidique de (c) ou le vecteur adénoviral ou l'adénovirus de (d).

2. Agent selon la revendication 1, dans lequel le gène LK68 comprend la séquence de nucléotides représentée par la SEQ ID NO : 1.

3. Agent selon la revendication 1, dans lequel le gène LK8 comprend la séquence de nucléotides représentée par la SEQ ID NO : 2.

4. Agent selon la revendication 1, dans lequel le vecteur rétroviral est pLXSN-LK8 ou pLXSN-LK68 illustré sur la Figure 1.

5. Agent selon la revendication 1, dans lequel le vecteur adéno-associé est pAAV-LK8 ou pAAV-LK68 illustré sur la Figure 10.

6. Agent selon la revendication 1, dans lequel l'ADN plasmidique est choisi dans l'ensemble constitué par pSecTag-LK8, pSecTag-LK68, pcDNA-LK8 et pcDNA-LK68.

7. Agent selon la revendication 1, dans lequel les cellules sont choisies dans l'ensemble constitué par les cellules souches hématopoïétiques, les cellules dendritiques, les cellules tumorales autologues et les cellules tumorales établies.

8. Agent selon la revendication 1, dans lequel le vecteur rétroviral de (a), le vecteur viral adéno-associé de (b), l'ADN plasmidique de (c) ou le vecteur adénoviral de (d) est présent à raison de 0,05 à 500 mg.

9. Agent selon la revendication 1, dans lequel le rétrovirus de (a), le virus adéno-associé de (b) ou l'adénovirus de (d) est présent à raison de 10³ à 10¹² UI.

10. Agent selon la revendication 1, dans lequel les cellules sont présentes à raison de 10³ à 10⁸ e.a.

11. Agent selon la revendication 1, dans lequel le cancer est choisi dans l'ensemble constitué par un carcinome du côlon, un cancer du foie, un cancer du poumon, un cancer du sein, un cancer du cerveau, un carcinome prostatique, un cancer de la peau, un cancer de l'estomac, un cancer du pancréas, un lymphome, un cancer du rein, un cancer ovarien et une tumeur métastasique.

12. Agent selon la revendication 11, dans lequel le cancer est choisi dans l'ensemble constitué par un carcinome du côlon, un cancer du foie, un lymphome et une tumeur métastasique.

13. Utilisation de l'agent pour thérapie génique selon l'une quelconque des revendications 1 à 12, dans la préparation d'un médicament pour la prévention ou le traitement d'une tumeur solide, dans laquelle le médicament est destiné à être administré par voie parentérale.

14. Utilisation de l'agent pour thérapie génique selon l'une quelconque des revendications 1 à 12, dans la préparation d'un médicament pour l'inhibition de la croissance et de la métastase d'une tumeur solide.

15. Utilisation selon la revendication 13 ou 14, dans laquelle le médicament est administré par injection hypodermique, injection hydrodynamique, injection intratumorale ou injection intramusculaire.
